(19) Europäisches Patentamt
European Patent Office
Office européen des brevets

(11) EP 0 775 133 B1

(12) **EUROPEAN PATENT SPECIFICATION**

(45) Date of publication and mention of the grant of the patent:
**30.05.2001 Bulletin 2001/22**

(21) Application number: **95929014.9**

(22) Date of filing: **27.07.1995**

(51) Int Cl.[7]: **C07D 401/12**, A61K 31/47, C07D 215/14, C07D 215/18

(86) International application number:
**PCT/EP95/02970**

(87) International publication number:
**WO 96/04267 (15.02.1996 Gazette 1996/08)**

# (54) NAPHTHALENE AMIDES HAVING LEUKOTRIENE-ANTAGONISTIC ACTION

NAPHTHALENAMIDE ALS LEUKOTRIEN ANTAGONISTEN

AMIDES DE NAPHTALENE A ACTION ANTAGONISTE DES LEUCOTRIENES

(84) Designated Contracting States:
**AT BE CH DE DK ES FR GB GR IE IT LI LU MC NL PT SE**

(30) Priority: **01.08.1994 ES 9401696**

(43) Date of publication of application:
**28.05.1997 Bulletin 1997/22**

(73) Proprietor: **LABORATORIOS MENARINI S.A.**
**08912 Badalona (ES)**

(72) Inventors:
- **MAULEON CASELLAS, David**
  **E-08912 Badalona (ES)**
- **CARGANICO, Germano**
  **E-08912 Badalona (ES)**
- **FOS TORRO, Maria de los Desamparados**
  **E-08912 Badalona (ES)**
- **GARCIA PEREZ, Maria Luisa**
  **E-08912 Badalona (ES)**
- **PALOMER BENET, Albert**
  **E-08912 Badalona (ES)**

(74) Representative: **Minoja, Fabrizio, Dr.**
**Bianchetti Bracco Minoja S.r.l.**
**Via Rossini, 8**
**20122 Milano (IT)**

(56) References cited:
**EP-A- 0 315 399** **EP-A- 0 348 155**
**US-A- 4 579 866** **US-A- 5 084 575**

- **JOURNAL OF MEDICINAL CHEMISTRY, vol. 35, no. 14, WASHINGTON US, pages 2501-2524, JOHN H. MUSSER ET AL. '5-Lipoxygenase: properties, ...'**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 775 133 B1

## Description

The present invention relates to novel naphthalene amides, the pharmaceutically acceptable salts and solvates thereof and pharmaceutical compositions containing them, having a leukotriene-antagonistic activity. The present invention also relates to a process for the preparation of the novel naphthalene amides, as well as to the therapeutic use thereof.

TECHNOLOGICAL BACKGROUND

It is well known that most eicosanoids, prostaglandins, leukotrienes and related compounds derive from a fatty acid having 20 carbons and 4 unsaturations, called arachidonic acid (AA), which fundamentally esterifies the hydroxyl at the 2- position of the glycerol of the phospholipids contained in the cell membranes. AA is released from the phospholipid containing it by the action of a lipase, phospholipase $A_2$ ($PLA_2$) ("CRC Handbook of Eicosanoids and Related Lipids", vol. II, Ed. A.L.Willis, CRS Press Inc., Florida (1989)). After being released AA is metabolized in mammals mainly by two different pathways or enzyme systems. Through cyclooxygenase it produces prostaglandins and thromboxanes, the most significant being $PGE_2$ and $TxA_2$, which are directly involved in inflammation (Higgs et al. Annals of Clinical Research, 16, 287 (1984)). Through lipo-oxygenase it produces leukotrienes, the most important being $LTB_4$, and the peptide-leukotrienes $LTC_4$, $LTD_4$ and $LTE_4$. All of them are also involved in inflammatory reactions, exhibiting chemotactic activities, stimulating the secretion of lysosomic enzymes and playing an important role in immediate hypersensitivity reactions (Bailey and Casey, Ann. Rep. Med. Chem., 17, 203 (1982)). Leukotriene $LTB_4$ is a strong chemotactic agent which promotes the infiltration of leukocytes and their subsequent degranulation. (Salmon et al., Prog. Drug Res., 37, 9 (1991)). It has been widely shown that $LTC_4$ and $LTD_4$ have strong constrictive action on human bronchi (Dahlen et al., Nature, 288, 484 (1980)), causing the obstruction of airways by inflammation and mucus production (Marom et al., Am. Rev. Resp. Dis., 126, 449 (1982)), being thus involved in the pathogenesis of bronchial asthma, chronic bronchitis, allergic rhinitis, etc. Peptide-leukotrienes also bring about a blood extravasation caused by the increase of vascular permeability (Camp et al., Br. J. Pharmacol., 80, 497 (1883)) and are involved in some inflammatory diseases such as atopic eczema and psoriasis. On the other hand, several effects of peptide-leukotrienes on human cardiovascular system have been observed; they are mainly involved in the pathogenesis of the ischaemic cardiopathy. This relationship has been confirmed by the fact that coronary arteries can produce these mediators (Piomelli et al., J. Clin. Res., 33, 521A (1985)). These effects, together with the strong contractions observed in heart tissue caused by $LTC_4$ and $LTD_4$, suggest that these mediators might contribute to other cardiovascular disorders, such as coronary spasm, heart anaphylaxis, cerebral oedema and endotoxic shock.

From what said above it follows that the control of the biological activity of leukotrienes through compounds which inhibit their release or antagonize their effects, represents a new rational approach to the prevention, elimination or improvement of different allergic, anaphylactic, inflammatory and thrombotic conditions, in which such mediators are involved.

In literature some compounds have been described that can be considered as structurally related to the compounds of the present invention, having an inhibiting action on enzyme 5-lypoxygenase, and a leukotriene antagonistic activity. Kreft A.F. et al. described 2-[6-(2-quinolinylmethoxy)-2-naphthyl]propionic acid (US 4,960,892) and derivatives thereof (US 5,084,575), among which there are compounds containing sulfonimide, hydroxamic acid or hydroxamate groups, but the amides of the present invention are not included in their general formulas.

On the other hand, Stevenson D. et al. (US 4,579,866) described amides having an inhibiting action on 5-lypoxygenase, but differing from the compounds of the present invention in two aspects: first, they contain a phenyl instead of a naphthyl; second, they contain an alkyl chain with only one carbon atom with or without branches between the amide function and the terminal carboxylic acid. In short, they are compounds containing a phenylalanine or glycine terminal residue.

EP-A-315399 and EP-A-348155 disclose quinoline derivatives having leukotriene antagonist: said derivatives are characterized by the presence of a phenylacetamide moiety instead of the naphthylacetamide of the compounds according to the invention.

The obtaining of compounds with high leukotriene antagonistic activity is still a problem in the therapy. The present invention provides a number of novel compounds that exhibit the above mentioned antagonistic action and that are useful in therapy.

DISCLOSURE OF THE INVENTION

The present invention relates to novel naphthalene amides of general formula **I**,

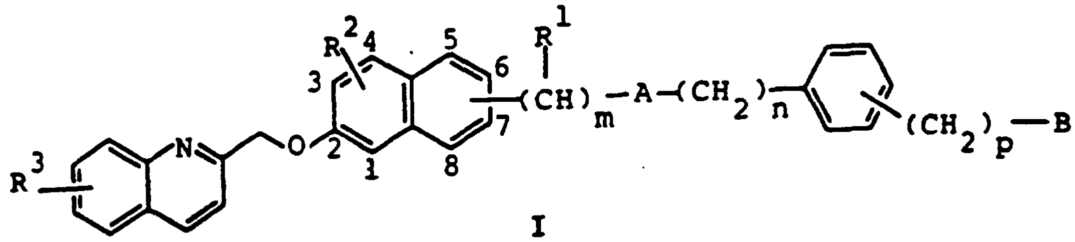

I

wherein:

the substituent containing A is bound to the 6- or 7-position of the 2-naphthol system;
the substituent containing B is bound to the benzene ring at any free position;
-$R^1$ is hydrogen or methyl;
-$R^2$ is hydrogen, fluorine, chlorine or -$OCH_3$, which is bound to the naphthalene system at any position except the 2- and the one occupied by the other substituent;
-$R^3$ is hydrogen, fluorine, chlorine or bromine;
-A- is a -CO-$NR^4$- or -$NR^4$-CO- group, wherein $R^4$ is hydrogen or methyl;
-B is a 5-tetrazolyl or -$COOR^5$ group, wherein $R^5$ is hydrogen, a ($C_1$-$C_4$)-alkyl or a phenylalkyl group of less than 10 carbon atoms;
m is 0 or 1;
n and p are integers from 0 to 6, with the proviso that n + p is less or equal to 6.

The present invention also relates to a process for the preparation of the novel naphthalene amides, as well as the therapeutical use thereof.

The present invention also relates to the solvates and the pharmaceutically acceptable salts of the amides of formula I and particularly the salts represented by formula **Ia**,

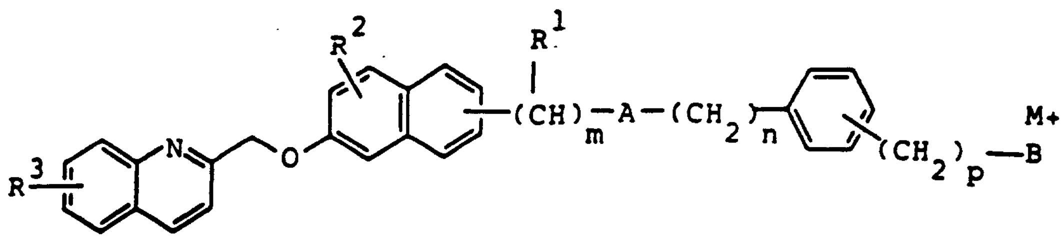

**Ia**

wherein $M^+$ is an alkali metal cation (e.g. $Na^+$, $K^+$) or represents a half amount of an alkaline-earth metal cation (e.g. 1/2 $Ca^{2+}$, 1/2 $Mg^{2+}$), or a cation deriving from an amine or quaternary ammonium salt (such as triethanolammonium, tris(hydroxymethyl)methylammonium).

The compounds of formula **I** can have one or more asymmetric carbons in their structure. The present invention comprises all the possible stereoisomers as well as the mixtures thereof.

Preferred compounds are those wherein $R^2$ is hydrogen and B is a 5-tetrazolyl or $COOR^5$ group, wherein $R^5$ is hydrogen, methyl, ethyl or benzyl.

Also preferred are the compounds of formula I wherein $R^3$ is hydrogen or chlorine and -A- is -CONH- or -NHCO-.

When the substituent containing A is bound to the 6- position of the 2-naphthol system, particularly preferred are the compounds of formula I wherein $R^1$ is hydrogen, m is 1, and -A- is -NHCO-; or those wherein -A- is -CONH-, being n and p integers ranging between 0 and 3.

When the substituent containing a A is bound to the 7- position of the 2-naphthol system, particularly preferred are the compounds of formula **I** wherein $R^1$ is hydrogen, m is 1 and -A- is -CONH-; or also those wherein m is 0, -A- is -CONH-, being n and p integers ranging between 0 and 3.

Particularly preferred compounds of the present invention are the following ones: N-[4-(1H-5-tetrazolyl)phenylmethyl]-2-[6-(2-quinolinylmethoxy)-2-naphthyl]propanamide;

N-[3-(1H-5-tetrazolyl)phenylmethyl]-2-[6-(2-quinolinylmethoxy)-2-naphthyl]propanamide;
N-[2-(1H-5-tetrazolyl)phenylmethyl]-2-[6-(2-quinolinylmethoxy)-2-naphthyl]propanamide;
N-[4-(1H-5-tetrazolyl)methylphenyl]-2-[6-(2-quinolinylmethoxy)-2-naphthyl]propanamide;
N-[4-(1H-5-tetrazolyl)methylphenyl]-2-[6-(2-quinolinylmethoxy)-2-naphthyl]propanamide (sodium salt);
4-[2-[6-(2-quinolinylmethoxy)-2-naphthyl]propanamido]benzoic acid;
4-[4-[2-[6-(2-quinolinylmethoxy)-2-naphthyl]propanamido]phenyl]butanoic acid;
4-[2-[6-(2-quinolinylmethoxy)-2-naphthyl]propanamidomethyl]benzoic acid;
3-[2-[6-(2-quinolinylmethoxy)-2-naphthyl]propanamidomethyl]benzoic acid;
4-[2-[6-(2-quinolinylmethoxy)-2-naphthyl]propanamido]phenylacetic acid;
3-[2-[6-(2-quinolinylmethoxy)-2-naphthyl]propanamido]phenylacetic acid;
4-[4-[2-[6-[(7-chloro-2-quinolinyl)methoxy]-2-naphthyl]propanamido]phenyl]butanoic acid;
N-[4-(1H-5-tetrazolyl)phenylmethyl]-6-(2-quinolinylmethoxy)-2-naphthaleneacetamide;
4-[4-[[6-(2-quinolinylmethoxy)-2-naphthyl]carboxamido]phenyl]butanoic acid;
N-[3-(1H-5-tetrazolyl)phenylmethyl]-6-(2-quinolinylmethoxy)-2-naphthalenecarboxamide;
4-[4-[[7-(2-quinolinylmethoxy)-2-naphthyl]carboxamido]phenyl]butanoic acid;
N-[4-(1H-5-tetrazolyl)phenylmethyl]-7-(2-quinolinylmethoxy)-2-naphthalenecarboxamide;
4-[2-[[7-(2-quinolinylmethoxy)-2-naphthyl]carboxamido]ethyl]benzoic acid;
N-[4-(1H-5-tetrazolyl)phenylethyl]-7-(2-quinolinylmethoxy)-2-naphthalenecarboxamide;
4-[4-[[6-(2-quinolinylmethoxy)-2-naphthyl]methylaminocarbonyl]phenyl]butanoic acid;
N-[4-(1H-5-tetrazolyl)phenylpropyl]-7-(2-quinolinylmethoxy)-2-naphthalenecarboxamide;

as well as the carboxylic acid esters described in the examples.

According to the present invention, the compounds of general formula I wherein A is -CO-$NR^4$- are obtained by a process in which, starting from a compound of general formula **II**,

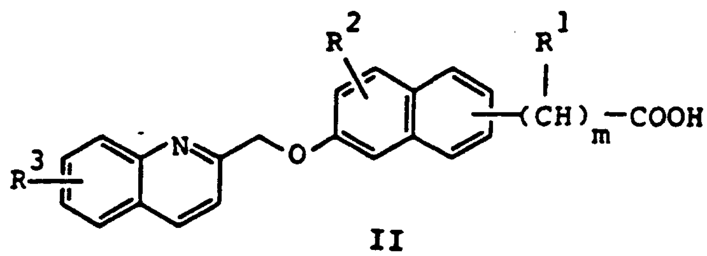

II

II

wherein $R^1$, $R^2$, $R^3$ and m have the above defined meanings, is reacted with a compound **III**,

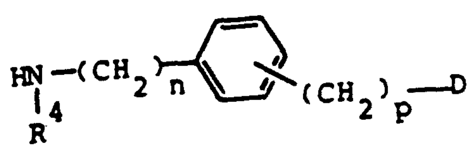

III

wherein $R^4$, n and p have the above defined meanings and D can be equivalent to the group B in **I** or, when B in formula **I** is COOH, then D contains a suitable carboxy-protecting group, for example as a methyl, ethyl or benzyl ester. The reaction between **II** and **III** is carried out in the presence of a carboxy-activating agent such as dicyclohexylcarbodiimide or 1-[3-(dimethylamino)propyl]-3-ethylcarbodiimide and a base such as triethylamine or 4-dimethylaminopyridine, in a suitable aprotic solvent such as chloroform, methylene chloride or N,N-dimethylformamide, at a temperature ranging between 0° and 40°C for a time from 3 to 24 hours. In this way, a compound of formula **IVa** is obtained,

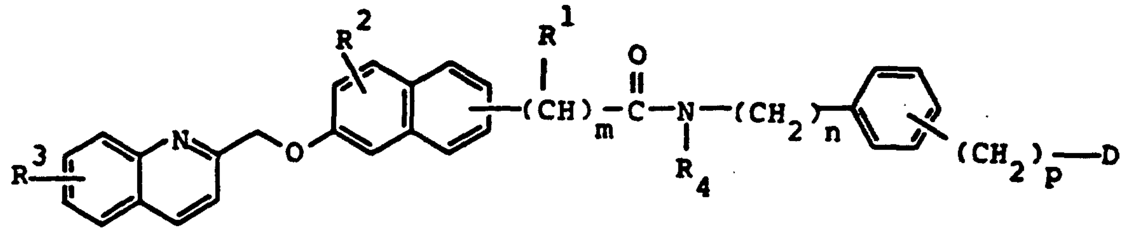

IVa

this compound coincides with **I** or is converted into **I** removing any COOH-protecting groups present in D, then, when D is for example a methyl, ethyl or benzyl ester, can be removed by treatment with a suitable base such as lithium or sodium hydroxide in aqueous solution, in a suitable organic solvent such as methanol, ethanol or tetrahydrofuran, at a temperature ranging between 20°C and the solvent reflux, for a time from 1 to 48 hours.

A compound of general formula **I** wherein A is $-NR^4-CO-$ is obtained according to the same process as above, starting from the compounds **V** and **VI**,

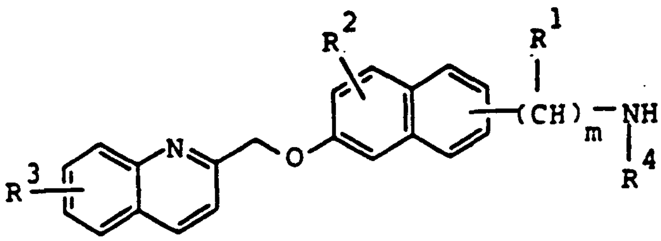

V

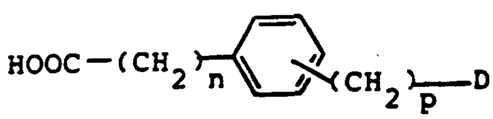

VI

wherein $R^1$, $R^2$, $R^3$, $R^4$, D, m, n and p have the above defined meanings. In this way a compound of formula **IVb**,

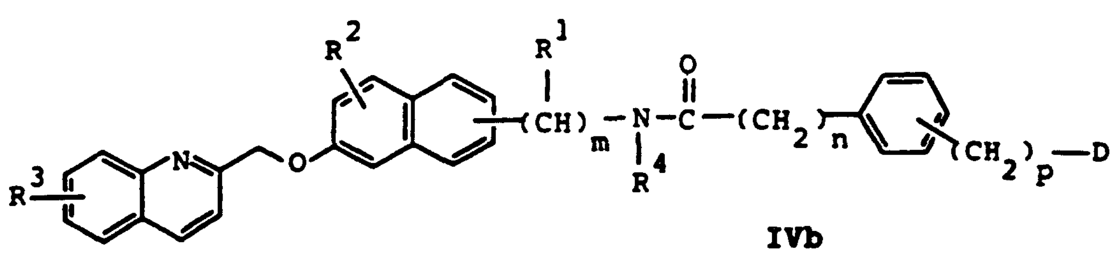

IVb

which coincides to **I** or is converted into **I** removing any COOH-protecting groups present in D, is obtained as described above.

When a given salt of general formula **Ia** is desired, a compound **I** can be treated with a suitable base or ion-exchanger, according to the conventional chemical techniques. Thus, for example, **I** can be treated with sodium hydroxide or tris(hydroxymethyl)methylamine in a suitable solvent such as water-methanol or ethanol mixtures, for a time from 15 min to 2 hours, at a temperature ranging between 25*C and the solvent reflux.

A starting product of formula **II** can be obtained, for example, following the synthesis sequence shown in scheme 1.

**Scheme 1**

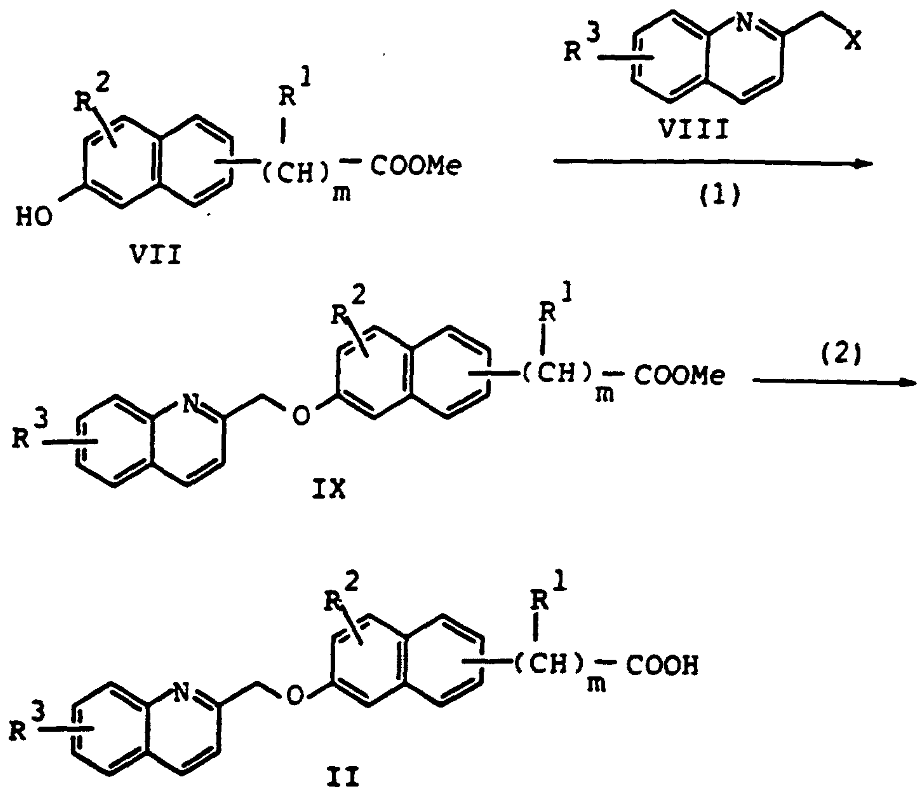


In this synthetic sequence, a compound **IX** can be obtained, for example, subjecting a compound **VII** to the action of a base such as sodium methoxide or sodium hydride, thereafter reacting it with a compound **VIII**, wherein $R^3$ represents the groups defined above and X is a bromine or chlorine atom, in a suitable organic solvent such as benzene, N,N-dimethylformamide or tetrahydrofuran, at a temperature ranging between 0° and 25°C for a time from 3 to 24 hours (step (1)).

A compound **II** can be obtained starting from **IX** (step (2)) by basic hydrolysis as described for the preparation of **I** with B=COOH starting from **IVa**.

Analogously, a starting compound of general formula **V** can be obtained, for example, following the synthetic sequence shown in scheme 2.

**Scheme 2**

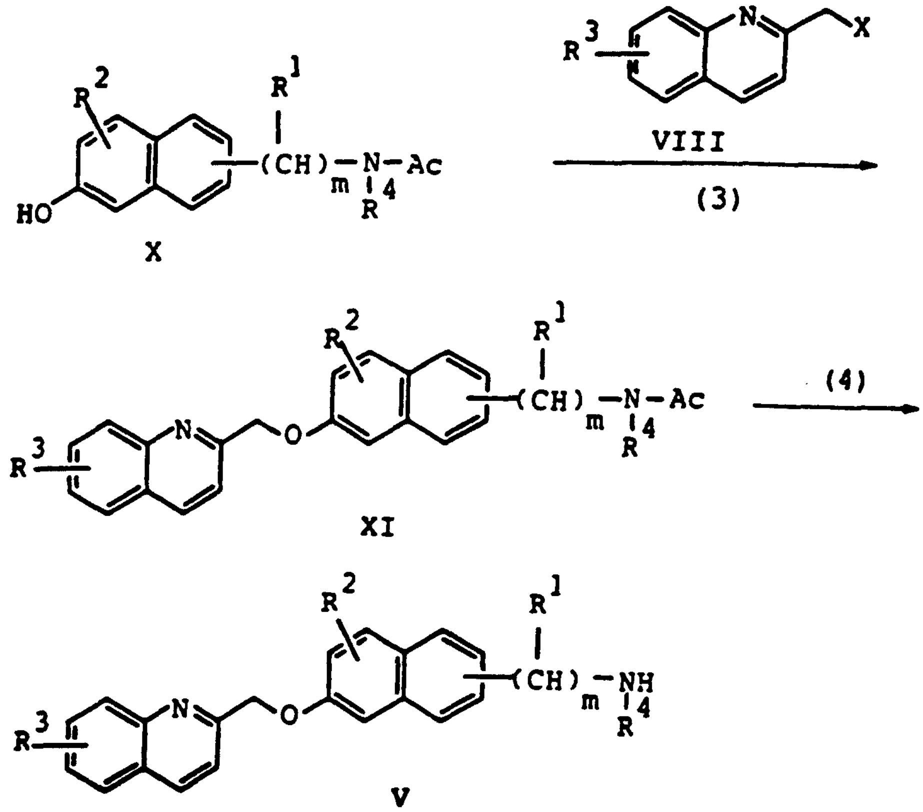


X

XI

V

In this synthetic sequence a compound **XI** is obtained starting from a compound **X** (step (3)), following the same process as described for the preparation of **IX.**

A compound **V** can be obtained starting from **XI** by hydrolysis with hydrochloric or sulfuric acid, in a suitable solvent such as tetrahydrofuran or dioxane, at a temperature ranging between 25° and the solvent reflux, for a time from 1 to 24 hours (step (4)).

A starting compound **VII** can be obtained, for example, following the synthetic sequence shown in scheme 3.

**Scheme 3**

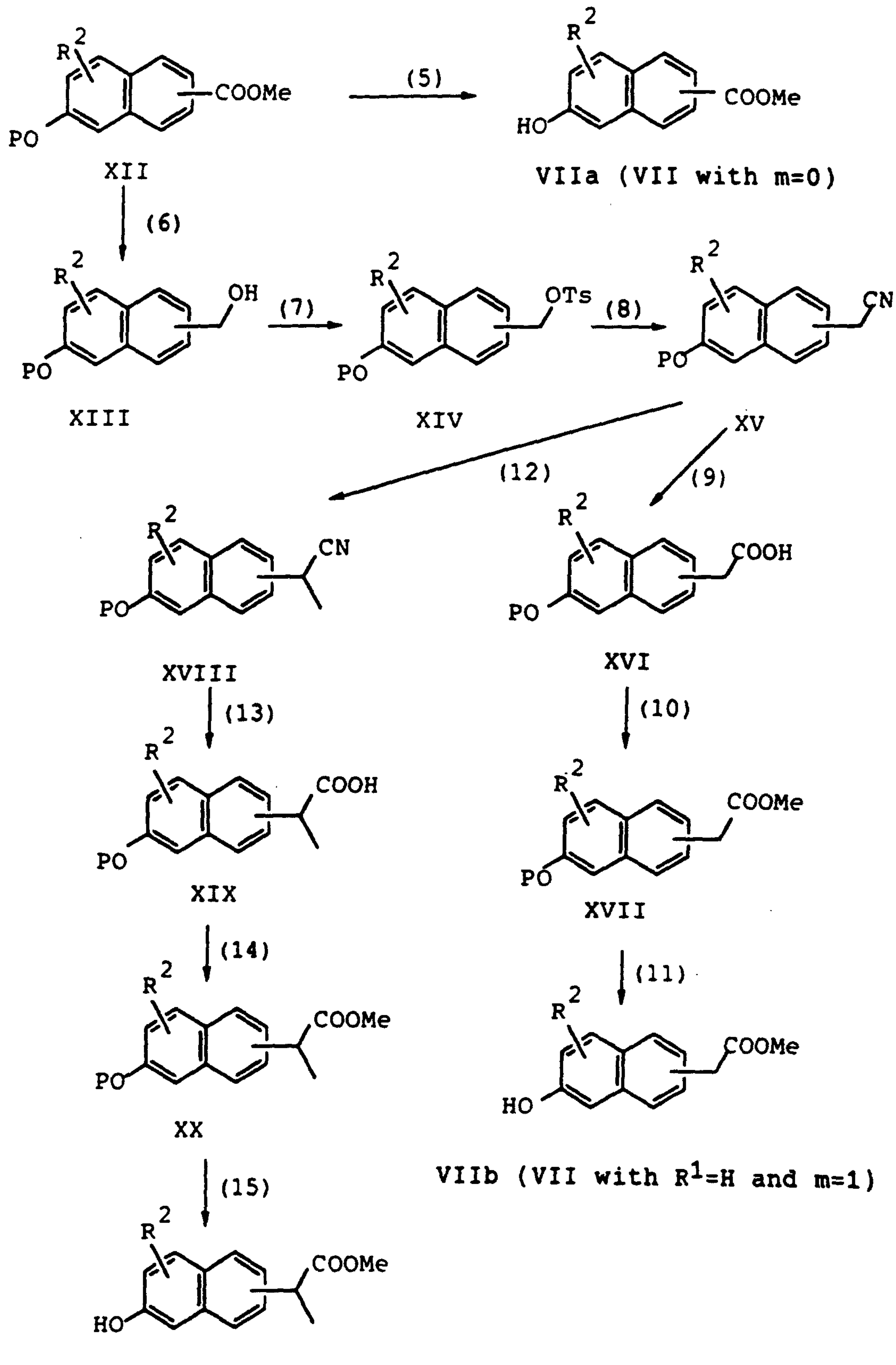


XII

VIIa (VII with m=0)

XIII

XIV

XV

XVIII

XVI

XIX

XVII

XX

VIIb (VII with $R^1$=H and m=1)

VIIc (VII with $R^1$=$CH_3$ and m=1)

In this sequence the starting product is a compound of general formula **XII**, wherein $R^2$ represents the groups described above and P represents a suitable hydroxy-protecting group, such as a methyl or t-butyldimethylsilyl group. Said compound can be obtained easily starting from the corresponding hydroxynaphthoic acids, following preparation processes described in literature (Gray G.W. et al., J. Chem. Soc., 678 10 (1954) and Daines et al., WO 9217172).

Starting from **XII, VIIa** (**VII** with m=0) can be obtained removing the protecting group P (step (5)); for example when this is a methyl group, it can be removed by means of boron tribromide in an organic solvent such as methylene chloride or chloroform, at a temperature ranging between -78° and 0°C for a time from 2 to 8 hours. When P is a t-butyldimethylsilyl group, it can be removed with a base such as potassium carbonate or bicarbonate, in a solvent such as tetrahydrofuran, methanol or dioxane at a temperature ranging between 0° and 50°C, for a time from 2 to 12 hours.

A compound **VIIb** (**VII** with $R^1$=H and m=1) can be obtained also starting from a compound **XII**. By means of a step (6), a compound **XII** is subjected to the action of a suitable metal hydride, such as sodium borohydride, in an solvent such as methanol, ethanol or tetrahydrofuran, in the presence of a catalytic amount of water, at a temperature ranging between 20°C and the solvent reflux, for a time from 3 to 24 hours: in this way compound **XIII** is obtained. This compound is subjected to a tosylation reaction (step (7)) with tosyl chloride in the presence of pyridine or triethylamine in methylene chloride, to give a compound of formula **XIV,** which is treated with sodium cyanide in tetrahydrofuran or DMSO at a temperature between 25°C and the solvent reflux to yield compound **XV** (step (8)). The hydrolysis of **XV**, for example with NaOH in ethanol at a temperature ranging between 25°C and the solvent reflux, followed by the esterification with methanol in the presence of sulfuric acid, gives compound **XVII** (steps (9) and (10) respectively). Finally, the removal of the protecting group P, as described above, can lead to a compound **VIIb.**

Analogously, a compound **VIIc** (**VII** with $R^1$=$CH_3$ and m=1) can be obtained by a synthesis sequence in which the starting product is a compound **XV.** By treating **XV** with a strong base, such as sodium methoxide or potassium t-butoxide, in a suitable solvent such as tetrahydrofuran or N,N-dimethylformamide, at a temperature ranging between -30° and 25°C for a time from 2 to 24 hours (step (12)), a compound **XVIII** is obtained. After that, by hydrolysis of the nitrile group present in **XVIII** (step (13)), esterification of the carboxylic group of **XIX** (step (14)) and elimination of the protecting group P in **XX** (step (15)), as described above, compound **VIIc** is obtained.

Alternatively, a compound **VIIb** can be obtained following the synthesis shown in scheme 4.

**Scheme 4**

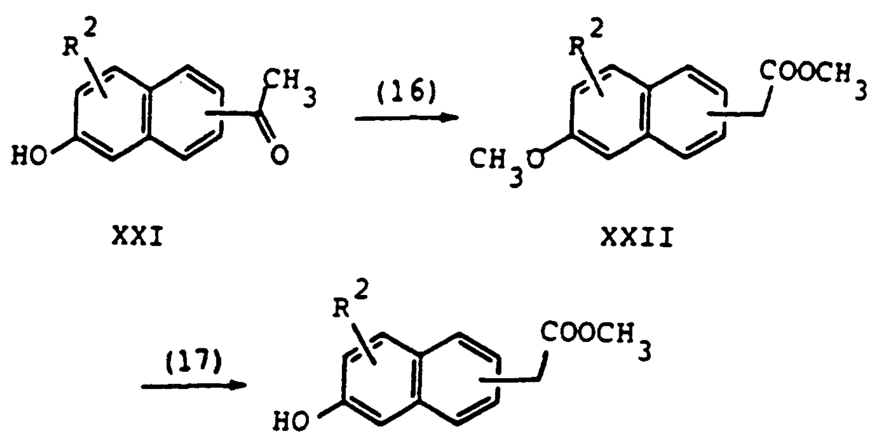


XXI

XXII

VIIb (VII with $R^1$=H and m=1)

Starting from a compound of general formula **XXI,** which is prepared easily following processes described in literature (Müller et al. Helv. Chim. Acta, 57, 790 (1974)), a compound **XXII** can be prepared by the modified Willgerodt's reaction. By means of such a reaction, treating a compound **XXI** with sulfur in the presence of an amine such as morpholine, which in its turn acts as the solvent, at the solvent reflux temperature, for a time from 6 to 24 hours, a compound of formula **XXII** (step (16)) is obtained after a suitable treatment with hydrochloric acid. The removal of the hydroxy-protecting methyl group, according to the process described above, leads to the preparation of a compound **VIIb** (step (17)).

Alternatively, a compound **VIId**, equivalent to **VII** with $R^1$=$CH_3$, $R^2$=H, m=1 and with an alkylmethoxycarbonyl substituent at the 6- position of the 2-naphthol system can be prepared, starting from a compound **XXIII**, which is the known NSAID naproxen, commercially available both as the racemate and in the form of the two resolved enantiomers.

Compound **XXIII** is subjected to demethylation with $BBr_3$ in a solvent such as chloroform or methylene chloride at a temperature between -78°C and room temperature for 1 to 6 hours, followed by addition of methanol to the reaction, to obtain the desired compound **VIId** (scheme 5).

**Scheme 5**

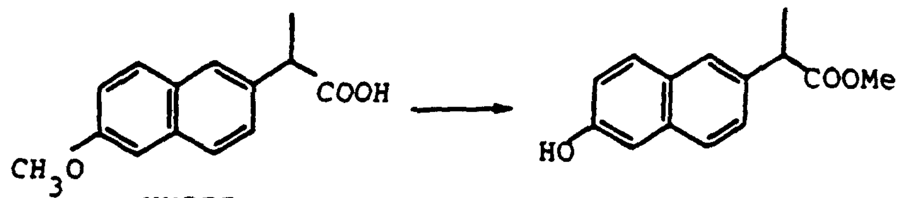

XXIII

VIId (VII with $R^1$=$CH_3$, $R^2$=H, m=1 and a methoxycarbonylalkyl substituent at the 6-position)

Alternatively a compound **IIa,** i.e. a compound **II** with m=0, can be obtained following the process described in scheme 6.

**Scheme 6**

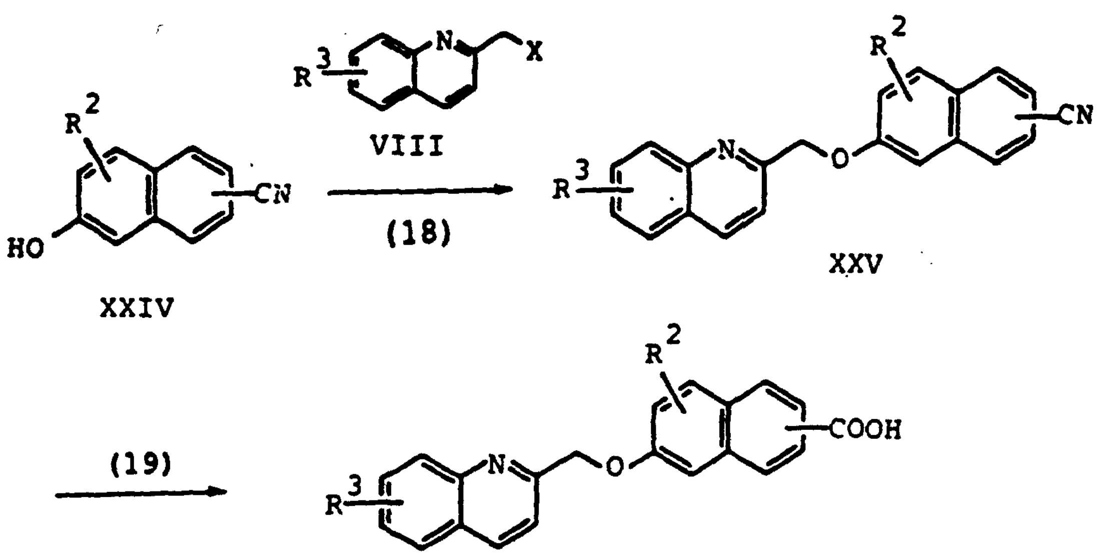


VIII

XXIV

XXV

IIa (II with m=0)

Starting from a compound **XXIV,** easily available following chemical processes described in literature (Tolbert et al. J. Am. Chem. Soc., 112. 8163 (1990)), a compound **XXV** can be prepared by condensation with **VIII** (step (18)) following the method described for the preparation of the compounds of general formula **IX.** The hydrolysis of **XXV** (step (19)), according to the process described for the preparation of **XIX**, leads to the compounds of general formula **IIa.**

A compound of general formula **Xa**, i.e. of general formula **X** with $R^1$=H, $R^4$=H and m=1, can be obtained, for example, following the synthetic sequence shown in scheme 7.

**Scheme 7**

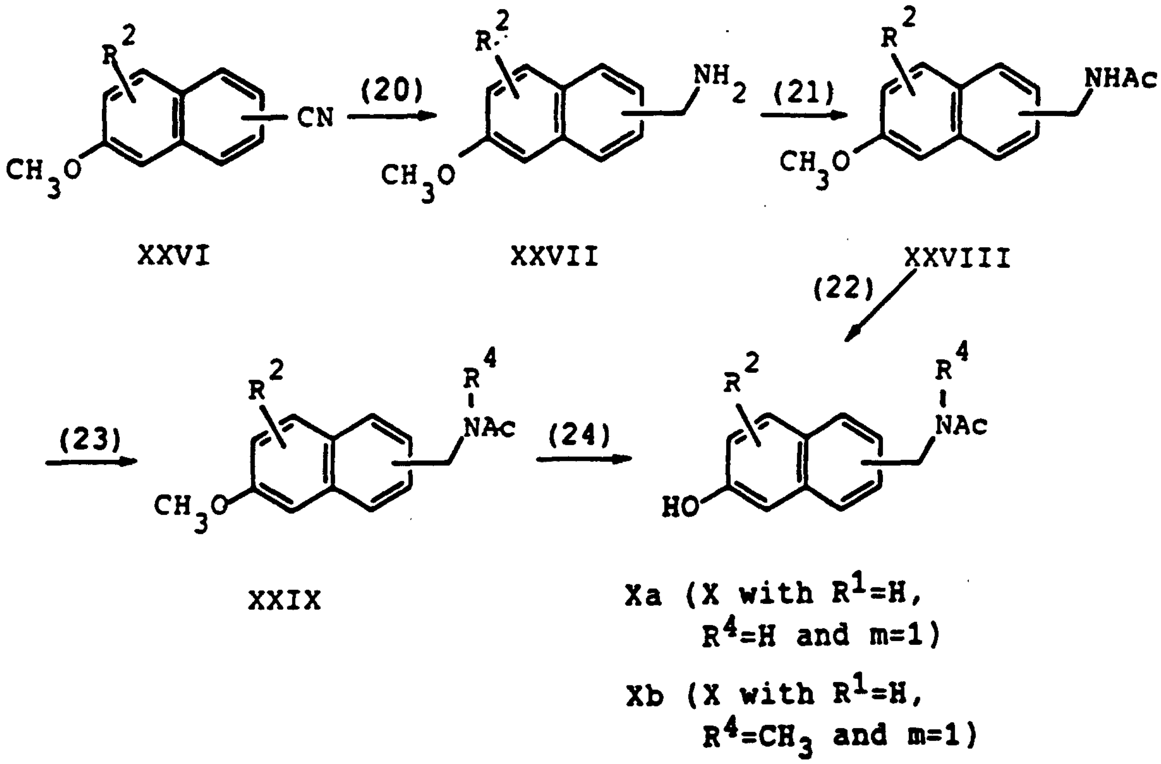


XXVI XXVII XXVIII

XXIX

Xa (X with $R^1$=H, $R^4$=H and m=1)

Xb (X with $R^1$=H, $R^4$=$CH_3$ and m=1)

A compound **XXVII** can be obtained starting from a compound of general formula **XXVI**, commercial or easily available starting from **XXIV**, by catalytic hydrogenation with a suitable catalyst, such as Pd-C or $Pd(OH)_2$-C in a suitable solvent such as ethanol or methanol, in the presence of an acid such as acetic acid or hydrochloric acid, under hydrogen pressures ranging between atmosphere pressure and 2 kg/$cm^2$, at a temperature from 25° to 50°C, for a time from 3 to 24 hours (step (20)).

A compound **XXVIII** can be obtained by acetylation of **XXVII** for example with acetic anhydride in the presence of a base such as pyridine or triethylamine in a solvent such as chloroform or methylene chloride, at a temperature between -78° and 25°C for a time from 1 to 6 hours (step (21)).

A compound **Xa** can be obtained starting from **XXVIII** by deprotecting the methylated hydroxy group (step (22)) according to the process described above. Analogously, a compound **Xb**, i.e. a compound of general formula **X** with $R^1$=H,, $R^4$=$CH_3$ and m=1, can be obtained by methylation of a compound **XXVIII** (step (23)) with methyl iodide or methyl sulfate in the presence of a base such as lithium diisopropylamide, lithium amide or potassium t-butoxide in a suitable solvent such as tetrahydrofuran, benzene or toluene at a temperature ranging between -78° and 25°C for 2-6 hours. The subsequent deprotection of the methylated hydroxy group leads to a compound **Xb** (step (24)).

A compound **Xc**, i.e. a compound of general formula **X** with $R^1$=$CH_3$ and m=1, can be obtained for example following the synthetic sequence shown in scheme 8.

**Scheme 8**

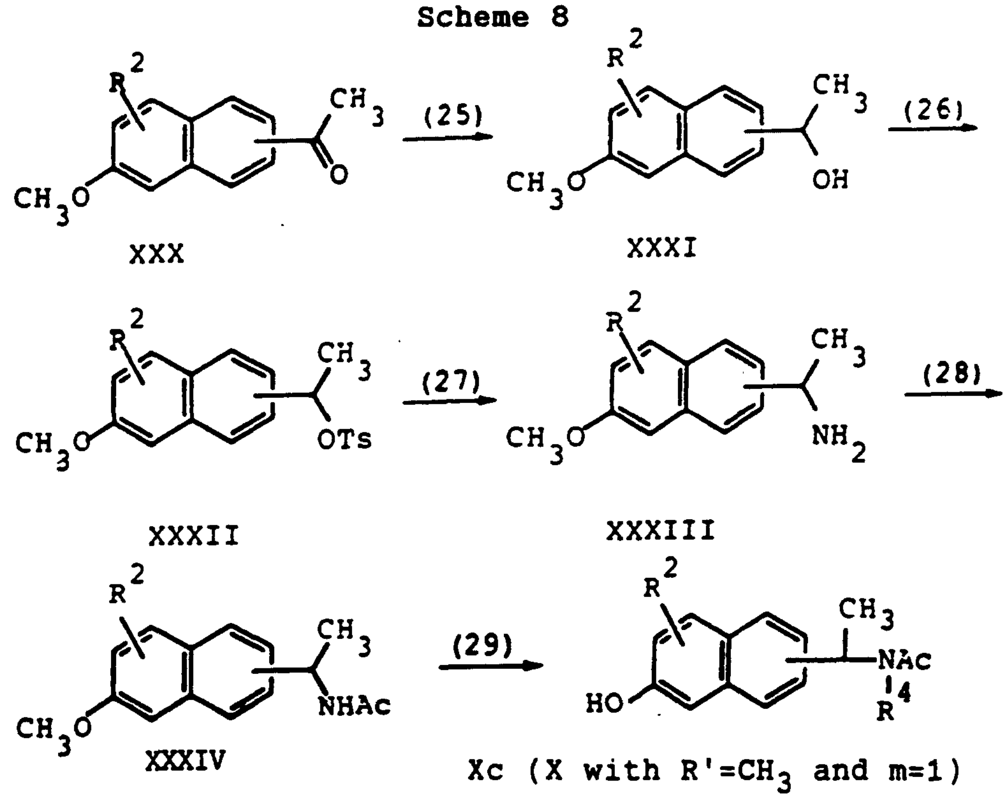


XXX

XXXI

XXXII

XXXIII

XXXIV

Xc (X with R'=$CH_3$ and m=1)

The reduction of a compound of formula **XXX** (easily available starting from **XXI**), for example, with a suitable hydride such as sodium borohydride in a solvent such as methanol, ethanol or tetrahydrofuran at a temperature between 20° and the solvent reflux for a time from 3 to 24 hours, gives a compound **XXXI** (step (25)).

A compound **XXXII** can be obtained, for example, by treatment of a compound **XXXI** (step (26)) with p-toluenesulfonic acid chloride in the presence of a base such as pyridine or triethylamine which can in their turn act as solvents, the presence of other solvents such as chloroform or methylene chloride being optional, at a temperature ranging between 20° and 40°C for a time from 3 to 18 hours.

A compound of formula **XXXIII** can be prepared for example starting from a compound **XXXII** (step (27)) by replacing the tosylate group with an azido group, using sodium azide as the reactive, in a suitable organic solvent such as N,N-dimethylformamide or tetrahydrofuran at a temperature ranging between 20°C and the solvent reflux for a time from 6 to 24 hours; and following catalytic hydrogenation of the intermediate azide with a suitable catalyst such as Pd-C or in a solvent such as ethanol, methanol or ethyl acetate, under hydrogen pressures ranging between atmosphere pressure and 2 kg/$cm^2$, at a temperature between 0° and 50°C, for a time from 6 to 24 hours.

Finally a compound **Xc** can be obtained by successive steps of acetylation, optional methylation of the amido group and deprotection of the methylated hydroxyl, as described above for the compounds **Xa** and **Xb**.

Analogously, a compound **Xd,** i.e. of general formula **X** with m=0, can be prepared starting from **XXXV**, easily available according to processes described in literature (Airan et al., J. Am. Chem. Soc., 28, 339 (1927)), by the process shown in scheme 9, in which a synthesis sequence similar to that described for the preparation of the other compounds **Xa-c** is used.

**Scheme 9**

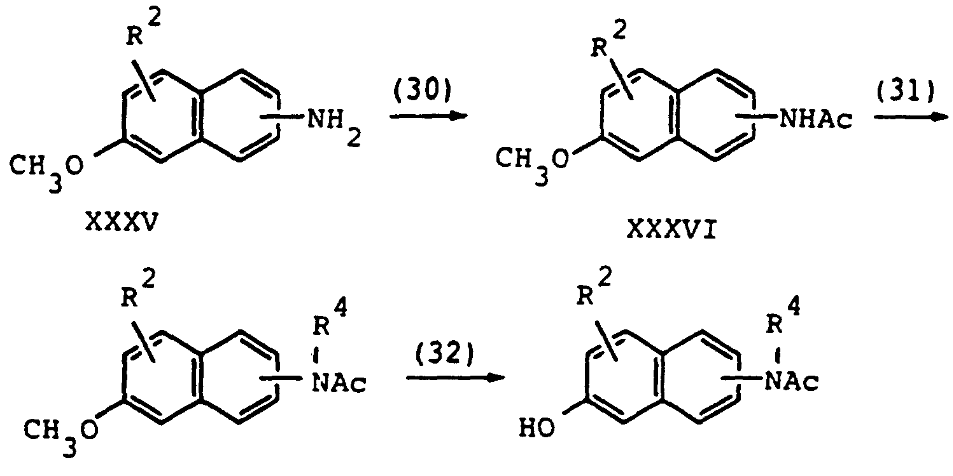


XXXV

XXXVI

XXXVII

Xd (X with m=0)

A starting compound **III** can be prepared following the synthesis sequence shown in scheme 10.

Scheme 10

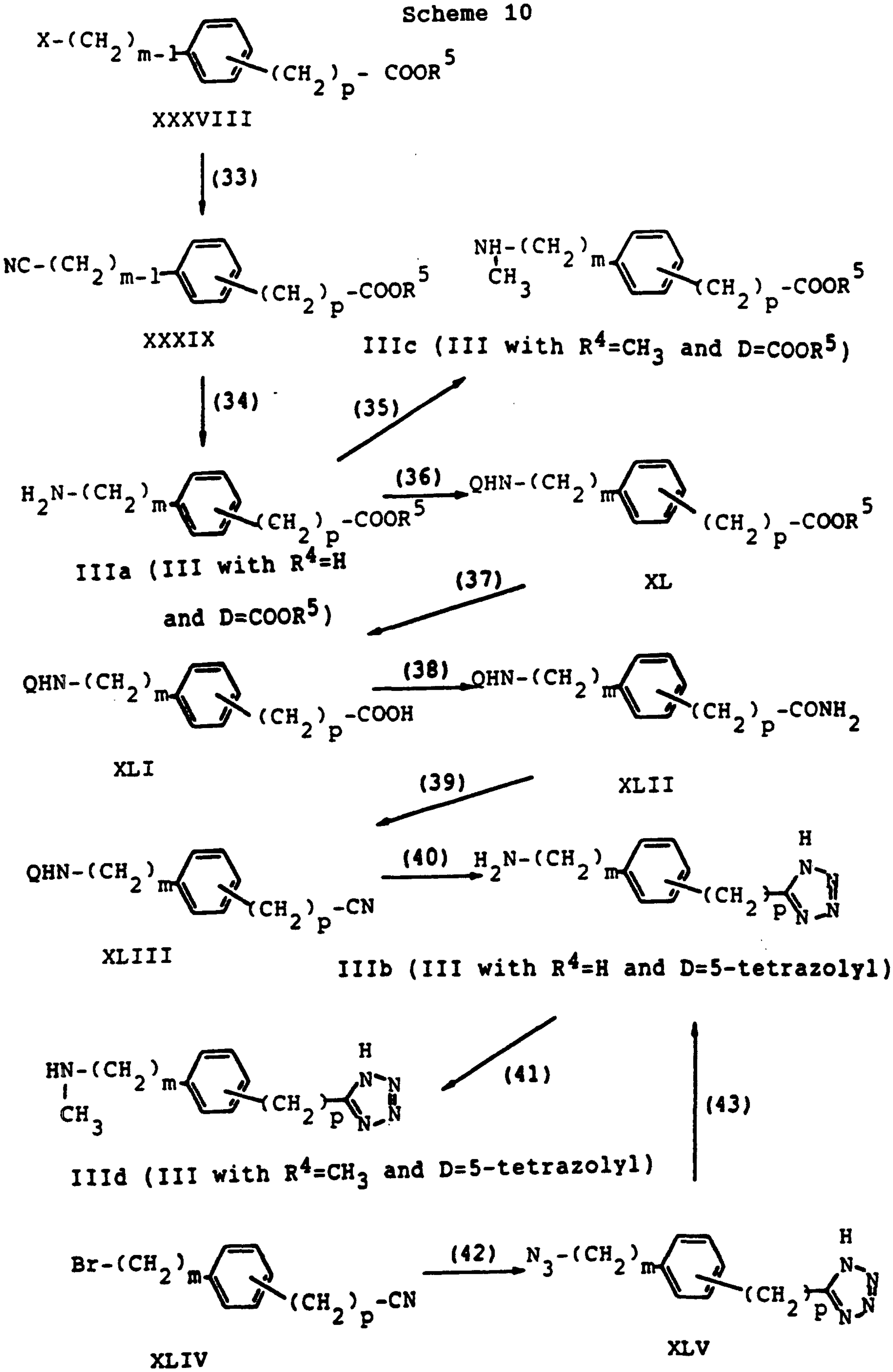


XXXVIII

XXXIX

IIIc (III with $R^4=CH_3$ and $D=COOR^5$)

IIIa (III with $R^4=H$ and $D=COOR^5$)

XL

XLI

XLII

XLIII

IIIb (III with $R^4=H$ and D=5-tetrazolyl)

IIId (III with $R^4=CH_3$ and D=5-tetrazolyl)

XLIV

XLV

Starting from a compound **XXXVIII**, commercial or easily available according to similar chemical processes, wherein m and p have the values described above, $R^5$ represents the groups described above, except hydrogen and X can be a halogen atom, a compound **XXXIX** can be prepared by treatment with NaCN in a suitable solvent such as dimethylsulfoxide, water or ethanol, at a temperature ranging between 25°C and the solvent reflux for 1-8 hours (step 33). By reduction of the cyano group in **XXXIX**, following the same process as described in step (20), a compound **IIIa** ca be prepared, which is equivalent to a compound of general formula **III** with $R^4$=H and D=COOR$^5$.

Starting from a compound **IIIa**, a compound **IIIb** can be obtained, i.e. of general formula **III** with $R^4$=H and D=5-tetrazolyl, by a process which comprises the steps (36)-(40). First, the amino group in **IIIa** is protected with a benzyloxycarbonyl or t-butoxycarbonyl group, according to processes widely described in literature, to yield compound **XL**, wherein Q represents one of the amino-protecting groups mentioned above. The basic hydrolysis of **XL** leads to the preparation of **XLI,** starting from which a compound **XLII** can be prepared by means, for example, of a reaction with ethyl chloroformate in the presence of a base such as triethylamine or pyridine in a solvent such as tetrahydrofuran or ethyl ether and following treatment with ammonia, at a temperature between 0° and 25°C, for a time from 30 min to 3 hours. The dehydration of a compound **XLII**, for example with phosphorous oxychloride, in a solvent such as N, N-dimethylformamide at a temperature ranging between 0° and 50°C for 3-24 hours, gives a compound **XLIII**. The treatment of a compound **XLIII** with sodium azide in a suitable solvent such as N,N-dimethylformamide at a temperature ranging between 25°C and the solvent reflux and the subsequent elimination of the protective group present in the amino group, according to conventional techniques, yields a compound **IIIb**. Alternatively, when a compound XLIV is commercial or easily available by similar synthetic methods, a compound **IIIb** can be obtained starting from **XLIV** by reaction with sodium azide (step (42)) and subsequent reduction of the azide (step 43), as described above in step (27).

Starting from a compound of formula **IIIa** or of formula **IIIb,** a compound **IIIc** or **IIId** can be obtained respectively by methylation of the amine according to, for example, a process comprising first the formylation of the amino group, with acetic anhydride and formaldehyde mixtures in a suitable solvent, such as tetrahydrofuran or ethyl ether, at a temperature between 0° and 25°C for 3-24 h, followed by reduction of the formyl group with the $BH_3$-tetrahydrofuran complex in a solvent such as tetrahydrofuran or ethyl ether at a temperature ranging between -78° and 0°C for 6-24 hours.

A starting compound of formula **VI** can be prepared for example following the process shown in scheme 11.

Starting from a compound **XLVI**, wherein m and p have the meanings described above and $R^5$ also represents the groups described above, except hydrogen, and Y represents a chlorine or bromine atom, commercially available in some instances or easily prepared by similar synthetic methods, **VIa,** i.e. the compound of general formula **VI** with D=COOR$^5$, (steps (44) and (45)), can be obtained following the processes described above for steps (33) and (13).

**Scheme 11**

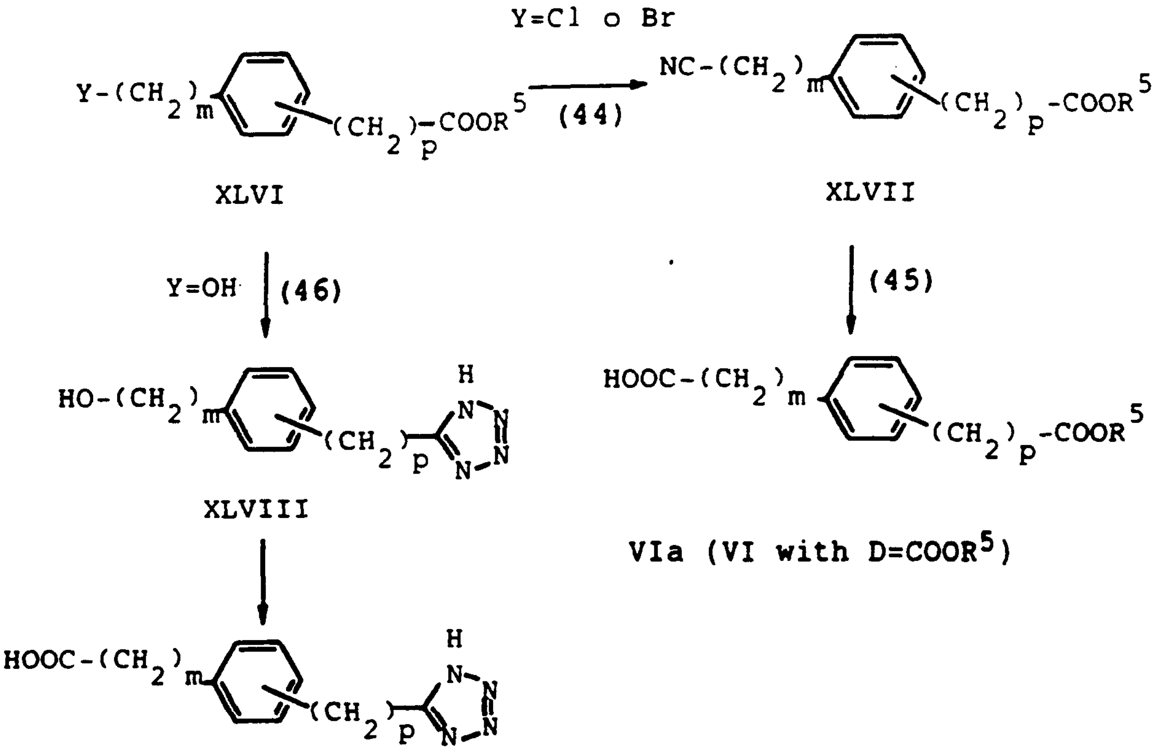


XLVI

XLVII

XLVIII

VIa (VI with D=$COOR^5$)

VIb (VI with D=5-tetrazolyl)

Starting from **XLVI**, wherein Y represents a hydroxyl group, a compound **XLVIII** can be prepared following a process similar to that described in scheme 10. Finally, a compound **VIb,** i.e. of general formula VI with D=5-tetrazolyl, can be prepared by a process which comprises the tosylation of **XLVIII,** the substitution of the tosylate by a nitrile group and the final hydrolysis according to the above described methods.

Alternatively, when in **VI** m=0, D=$COOR^5$ and the two substituents of the benzene ring are in para position, compopund of formula **VIc** can be prepared following the synthesis shown in scheme 12.

**Scheme 12**

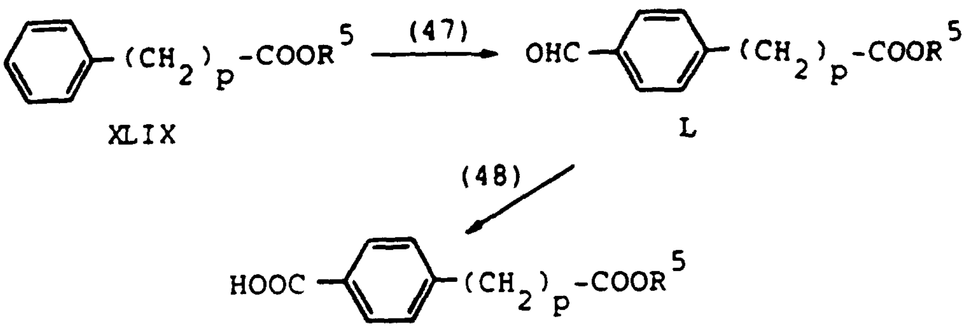


XLIX

L

VIc (VI with m=0, D=$COOR^5$ and para orientation of the substituents)

A compound **L** can be prepared starting from **XLIX**, for example, by a formylation reaction using a suitable

reactive such as hexamethylenetetramine or N,N-dimethylformamide in the presence of trifluoroacetic acid or phosphorous oxychloride at a temperature ranging between 25° and 100°C, for a time from 2 to 24 hours. The subsequent oxidation with a suitable oxidizing agent such as Jones's reagent at a temperature ranging between 0° and 25°C for a time of 2 to 18 hours, leads to the desired compound **VIc**.

The compounds of the present invention show a marked antagonistic activity of leukotrienes effects and they have therefore anti-inflammatory and anti-allergic properties which make them useful in the treatment of diseases wherein those mediators are involved.

Said compounds can be therefore used in human therapy, for the prevention and treatment of allergic rhinitis, bronchial asthma, hypersensitivity reactions such as allergic conjunctivitis, various inflammatory conditions such as rheumatoid arthritis, osteoarthritis, tendinitis, bursitis, psoriasis and related inflammations.

The compound of the present invention may also be used in the treatment of diseases of the cardiovascular system, such as cardiac ischemia, myocardic infarct, coronary spasm, cardiac anaphylaxis, cerebral oedema and endotoxic schock.

For the intended therapeutic uses, the compounds of the invention are formulated in suitable pharmaceutical compositions, using conventional techniques and methods, as disclosed in Remington's Pharmaceutical Science Handbook, Mack Pub. Co., N.Y. U.S.A. Examples of said formulations include capsules, tablets, syrups and the like, containing from 1 to 1000 mg of active principle per unit dose.

## EXAMPLES

The following examples illustrate the preparation and the pharmacological activity of the compounds of the present invention.

### Example 1: N-[4-(1H-5-tetrazolyl)phenylmethyl]-2-[6-(2-quinolinylmethoxy)-2-naphthyl]propanamide

#### 1A Methyl 2-(6-hydroxy-2-naphthyl)propionate

Boron tribromide (70 ml, 737 mmol) was added at -78°C to a solution of 2-(6-methoxy-2-naphthyl)propionic acid (10 g, 43.4 mmol) in dry methylene chloride (130 ml). The reaction mixture was stirred at room temperature for 5 h, then 136 ml of methanol were added and stirred for 18 hours. After this time, the mixture was evaporated to dryness, water (250 ml) was added and extracted with ethyl ether (4x100 ml). The combined ether phases were dried and the solvent was evaporated off, to obtain a crude which was purified by flash chromatography through a silica gel column. Eluting with petroleum ether:ethyl ether, 9:1, 6.9 g of the title compound were obtained as a white solid with melting point 184-186°C (70% yield).
$^1$H N.M.R. (300 MHz, $CDCl_3$) δ ppm: 1.56 (d, 3H); 3.70 (s, 3H); 3.87 (q, 1H); 5.75 (s, 1H); 7.08 (m, 2H); 7.38 (dd, 1H); 7.60 (d, 1H); 7.66 (m, 2H).

#### 1B Methyl 2-[6-(2-quinolinylmethoxy)-2-naphthyl]propionate

Sodium methoxide (6.0 ml, 31.2 mmol) was added to a solution of methyl 2-(6-hydroxy-2-naphthyl)propionate (7.2 g, 31.2 mmol) in DMF (200 ml) and stirred at room temperature for 5 min. After that 2-chloromethylquinoline (5.5 g, 31.2 mmol) was added thereto and the reaction mixture was stirred at room temperature for 18 h, then evaporated to dryness, the residue was dissolved in ethyl acetate (250 ml), washed with 5% $NaHCO_3$ (3x25 ml), dried and the solvent was evaporated off, to obtain a crude which was purified by crystallization with methanol. 9.3 g of the title compound wre obtained as a white solid with melting point 98-99°C (80% yield).
$^1$H N.M.R. (300 MHz, DMSO) δ ppm: 1.42 (d, 3H); 3.53 (s, 3H); 3.87 (q, 1H); 5.44 (s, 2H); 7.30 (m, 2H); 7.39 (d, 1H); 7.56 (t, 1H); 7.67 (m, 5H); 7.93 (d, 1H); 7.99 (d, 1H); 8.37 (d, 1H).

#### 1C 2-[6-(2-Quinolinylmethoxy)-2-naphthyl]propionic acid

1M lithium hydroxide (31.7 ml) was added to a solution of methyl 2-[6-(2-quinolinylmethoxy)-2-naphthyl]propionate (5 g, 13.2 mmol) in THF (40 ml) and stirred at room temperature for 48 h. After that THF was evaporated off, pH was adjusted to 4-5 with 1M HCl and the mixture was extracted with ethyl acetate, to obtain 4.7 g of the title compound as a white solid with melting point 192-194°C (99% yield).
$^1$H N.M.R. (300 MHz, DMSO) δ ppm: 1.39 (d, 3H); 3.76 (q, 1H); 5.44 (s, 2H); 7.29 (dd, 1H); 7.35 (dd, 1H); 7.40 (d, 1H); 7.58 (t, 1H); 7.69 (m, 3H); 7.77 (dt, 1H); 7.80 (d, 1H); 7.95 (d, 1H); 7.98 (d, 1H); 8.38 (d, 1H).

1D 4-(1H-5-Tetrazolyl)azidomethylbenzene

Sodium azide (15 g, 230 mmol) and ammonium chloride (12.3 g, 230 mmol) were added to a solution of 4-bromomethylbenzonitrile (5 g, 25.5 mmol) in DMF (75 ml). The reaction mixture was stirred at 110°C for 16 h, then poured onto 200 ml of 1M HCl and extracted with ethyl acetate (4x75 ml). The organic phase was dried and solvents were removed, to obtain a oil which was diluted with ethyl ether and petroleum ether, to give a precipitate which was filtered and washed with petroleum ether. In this way 5 g of the title compound were obtained (97% yield).
$^1$H N.M.R. (300 MHz, $CD_3OD$) δ ppm: 4.52 (s, 2H); 7.59 (d, 2H); 8.07 (d, 2H).

1E 4-(lH-5-Tetrazolyl)benzylamine hydrochloride

1.4 g of 10% palladium on charcoal was added to a solution of 4-(1H-5-tetrazolyl)azidomethyl-benzene (4.3 g, 21.5 mmol) in methanol (400 ml) and concentrated HCl (14 ml) and stirred at room temperature for 4 days, under hydrogen atmosphere. After that the reaction mixture was filtered and the filtrate was evaporated to dryness to obtain a crude which was redissolved in hot methanol, crystallizing in this way 3.7 g of the title compound as a yellowish solid with melting point >360°C (80% yield).
$^1$H N.M.R. (300 MHz, DMSO) δ ppm: 4.16 (d, 2H); 7.75 (d, 2H); 8.18 (d, 2H); 8.56 (s, 3H).

1F N-[4-(1H-5-Tetrazolyl)phenylmethyl]-2-[6-(2-quinolinylmethoxy)-2-naphthyl]propanamide

4-(lH-5-tetrazolyl)benzylamine hydrochloride (0.212 g, 1.0 mmol), N,N-dimethylaminopyridine (0.373 g, 3.05 mmol) and 1-[3-(dimethylamino)propyl]-3-ethylcarbodiimide hydrochloride (0.202 g, 1.05 mmol) were added in succession to a solution of 2-[6-(2-quinolinylmethoxy)-2-naphthyl]propionic acid (0.376 g, 1.05 mmol) in dry methylene chloride (50 ml). The reaction mixture was stirred at room temperature for 24 h, thereafter was poured onto water (30 ml) and pH was adjusted to 5 with 1M HCl, the phases were separated and the organic one was extracted with ethyl acetate (4x50 ml). The combined organic extracts were dried and the solvent was evaporated off, to obtain a crude which was purified by crystallization in methanol-chloroform-acetic acid mixtures, thereby obtaining 0.351 g of the title compound as a white solid with melting point 214-215°C (65% yield).
$^1$H N.M.R. (300 MHz, DMSO) δ ppm: 1.45 (d, 3H); 3.83 (q, 1H); 4.33 (d, 2H); 5.49 (s, 2H); 7.30 (dd, 1H); 7.35 (d, 2H); 7.44 (d, 1H); 7.47 (dd, 1H); 7.63 (dt, 1H); 7.72 (s, 1H); 7.78 (m, 4H); 7.92 (d, 2H); 8.01 (d, 1H); 8.05 (d, 1H); 8.44 (d, 1H); 8.61 (t, 1H).

Example 2: N-[3-(1H-5-tetrazolyl)phenylmethyl]-2[6-(2-quinolinylmethoxy)-2-naphthyl]propanamide

Following the process described in example 1 (point F), starting from 2-[6-(2-quinolinylmethoxy)-2-naphthyl] propionic acid and 3-(1H-5-tetrazolyl)benzylamine hydrochloride, the title compound was prepared as a white solid with melting point 114-115°C (70% yield).
$^1$H N.M.R. (300 MHz, DMSO) δ ppm: 1.48 (d, 3H); 3.86 (q, 1H); 4.38 (m, 2H); 5.52 (s, 2H); 7.33 (dd, 1H); 7.47 (m, 4H); 7.66 (dt, 1H); 7.80 (m, 6H); 7.98 (s, 1H); 8.04 (d, 1H); 8.09 (d, 1H); 8.47 (d, 1H); 8.66 (t, 1H).

Example 3: N-[2-(1H-5-tetrazolyl)phenylmethyl]-2-[6-(2-quinolinylmethoxy)-2-naphthyl]propanamide

Following the process described in example 1 (point F) starting from 2-[6-(2-quinolinylmethoxy)-2-naphthyl] propionic acid and 2-(1H-5-tetrazolyl)benzylamine hydrochloride, the title compound was prepared as a yellowish solid with melting point 189-190°C (57% yield).
$^1$H N.M.R. (300 MHz, DMSO) δ ppm: 1.40 (d, 3H); 3.80 (q, 1H); 4.52 (d, 2H); 5.49 (s, 2H); 7.35 (m, 6H); 7.63 (dt, 1H); 7.76 (m, 6H); 8.01 (dd, 1H); 8.05 (d, 1H); 8.43 (d, 1H); 8.49 (t, 1H).

Example 4: N-(4-cyanomethylphenyl)-2-[6-(2-quinolinylmethoxy)-2-naphthyl]propanamide

Following the process described in example 1 (point F), starting from 2-[6-(2-quinolinylmethoxy)-2-naphthyl] propionic acid and 4-cyanomethylaniline, the title compound was prepared as a white solid (recrystallized from methanol) with melting point 177-178°C (77% yield).
$^1$H N.M.R. (300 MHz, DMSO) δ ppm: 1.45 (d, 3H); 3.90 (m, 3H); 5.43 (s, 2H); 7.20 (d, 2H); 7.27 (dd, 1H); 7.39 (d, 1H); 7.46 (d, 1H); 7.56 (m, 3H); 7.69 (t, 2H); 7.48 (m, 2H); 7.81 (d, 1H); 7.94 (d, 1H); 8.00 (d, 1H); 8.36 (d, 1H); 10.13 (s, 1H).

Example 5: N-[4-(1H-5-tetrazolyl)methylphenyl]-2-[6-(2-quinolinylmethoxy)-2-naphthyl]propanamide

Following the process described in example 1 (point D), starting from N-(4-cyanomethylphenyl)-2-[6-(2-quinolinylmethoxy)-2-naphthyl]propanamide, the title compound was prepared as a white solid (recrystallized from methanol-ethyl acetate) with melting point 179.1-180.1°C (88% yield). $^1$H N.M.R. (300 MHz, DMSO) δ ppm: 1.47 (d, 3H); 3.93 (q, 1H); 4.20 (s, 2H); 5.47 (s, 2H); 7.16 (d, 2H); 7.30 (dd, 1H); 7.42 (d, 1H); 7.49 (dd, 1H); 7.55 (d, 2H); 7.60 (dt, 1H); 7.75 (m, 4H); 7.83 (d, 1H); 7.97 (d, 1H); 8.03 (d, 1H); 8.40 (d, 1H); 10.11 (s, 1H).

Example 6: N-[4-(1H-5-tetrazolyl)methylphenyl]-2-[6-(2-quinolinylmethoxy)-2-naphthyl]propanamide (sodium salt)

1M sodium hydroxide (0.31 ml) was added to a solution of N-[4-(1H-5-tetrazolyl)methylphenyl]-2-[6-(2-quinolinylmethoxy)-2-naphthyl]propanamide (0.161 g, 0.31 mmol) in methanol (5 ml) and stirred at room temperature for 30 min. After that the reaction mixture was evaporated to dryness and the residue was recrystallized from ethanol-ethyl ether, to obtain 0.143 g of the title compound as a white solid with melting point 278-279°C (85% yield).
$^1$H N.M.R. (300 MHz, DMSO) δ ppm: 1.47 (d, 3H); 3.93 (m, 3H); 5.47 (s, 2H); 7.16 (d, 2H); 7.30 (dd, 1H); 7.42 (d, 1H); 7.49 (dd, 1H); 7.55 (d, 2H); 7.60 (dt, 1H); 7.75 (m, 4H); 7.83 (d, 1H); 7.97 (d, 1H); 8.03 (d, 1H); 8.40 (d, 1H); 10.11 (s, 1H).

Example 7: methyl 4-[2-[6-(2-quinolinylmethoxy)-2-naphthyl]propanamidolbenzoate

Following the process described in example 1 (point F), starting from 2-[6-(2-quinolinylmethoxy)-2-naphthyl] propionic acid and methyl 4-aminobenzoate, the title compound was prepared as a white solid with melting point 182.0-182.3°C (72% yield). $^1$H N.M.R. (300 MHz, $CDCl_3$) δ ppm: 1.58 (dd, 3H); 3.81 (m, 4H); 5.43 (s, 2H); 7.18 (s, 1H); 7.24 (m, 1H); 7.35 (dd, 1H); 7.50 (m, 3H); 7.75 (m, 8H); 8.10 (d, 1H); 8.17 (d, 1H); 10.41 (s, 1H).

Example 8: 4-[2-[6-(2-quinolinylmethoxy)-2-naphthyl]propanamido]benzoic acid

Following the process described in example 1 (point C), starting from methyl 4-[2-[6-(2-quinolinylmethoxy)-2-naphthyl]propanamido]benzoate, the title compound was prepared as a white solid (recrystallized from ethanol) which'decomposes at 264-265°C (74% yield).
$^1$H N.M.R. (300 MHz, DMSO) δ ppm: 1.49 (d, 3H); 4.00 (q, 1H); 5.48 (s, 2H); 7.32 (dd, 1H); 7.42 (d, 1H); 7.58 (d, 1H); 7.75 (m, 10H); 7.99 (d, 1H); 8.04 (d, 1H); 8.42 (d, 1H); 10.41 (s, 1H).

Example 9: methyl 4-[4-[2-[6-(2-quinolinylmethoxy)-2-naphthyl]propanamidolphenyl]butanoate

9A Methyl 4-(4-aminophenyl)butanoate

8.4 ml of concentrated $H_2SO_4$ was added to a solution of 4-(4-aminophenyl)butanoic acid (2.0 g, 11.6 mmo) in methanol (84 ml) and refluxed for 2 h. After that the mixture was left to cool at room temperature, added with $Na_2CO_3$ to basic pH and extracted with ethyl acetate. The organic phase was dried and the solvent was evaporated off, to obtain 1.7 g of the title compound as a colourless oil (82% yield).
$^1$H N.M.R. (300 MHz, $CD_3OD$) δ ppm: 1.83 (q, 2H); 2.28 (t, 2H); 2.49 (t, 2H); 3.62 (s, 3H); 6.66 (d, 2H); 6.92 (d, 2H).

9B Methyl 4-[4-[2-[6-(2-quinolinylmethoxy)-2-naphthyl]propanamido]phenyl]butanoate

Following the process described in example 1 (point F), starting from 2-[6-(2-quinolinylmethoxy)-2-naphthyl] propionic acid and methyl 4-(4-aminophenyl)butanoate, the title compound was prepared as a white solid with melting point 145.7-149.0°C (87% yield).
$^1$H N.M.R. (300 MHz, $CDCl_3$) δ ppm: 1.61 (d, 3H); 1.82 (q, 2H); 2.27 (t, 2H); 2.56 (t, 2H); 3.62 (s, 3H); 3.81 (m, 1H); 5.42 (s, 2H); 7.02 (d, 2H); 7.28 (dd, 1H); 7.37 (m, 3H); 7.70 (m, 7H); 7.81 (d, 1H); 8.12 (d, 1H); 8.19 (d, 1H).

Example 10: 4-[4-[2-[6-(2-quinolinylmethoxy)-2-naphthyl]propanamido]phenyl]butanoic acid

Following the process described in example 1 (point C), starting from methyl 4-[4-[2-[6-(2-quinolinylmethoxy)-2-naphthyl]propanamido]phenyl]butanoate, the title compound was prepared as a white solid with melting point 176.0-176.3°C (68% yield).
$^1$H N.M.R. (300 MHz, DMSO) δ ppm: 1.42 (d, 3H); 1.68 (q, 2H); 2.12 (t, 2H); 2.45 (t, 2H); 3.97 (q, 1H); 5.55 (s, 2H); 7.03 (d, 2H); 7.26 (dd, 1H); 7.38 (d, 1H); 7.48 (m, 3H); 7.57 (t, 1H); 7.72 (m, 5H); 7.95 (d, 1H); 8.00 (d, 1H); 8.38 (d, 1H).

Example 11: methyl 4-[2-[6-(2-quinolinylmethoxy)-2-naphthyl]propanamidomethyl]benzoate

11A Methyl 4-cyanobenzoate

Following the process described in example 9 (point A), starting from 4-cyanobenzoic acid, the title compound was prepared as a yellowish oil (93% yield).
$^1$H N.M.R. (300 MHz, $CDCl_3$) δ ppm: 4.11 (s, 3H); 7.89 (d, 2H); 8.28 (d, 2H).

11B Methyl 4-aminomethylbenzoate

Following the process described in example 1 (point E), starting from methyl 4-cyanobenzoate and reacting for 4 h, the title compound was prepared as a semi-solid oil (87% yield).
$^1$H N.M.R. (300 MHz, $CD_3OD$) δ ppm: 3.91 (s, 3H); 4.21 (s, 2H); 7.59 (d, 2H); 8.07 (d, 2H).

11C Methyl 4-[2-[6-(2-quinolinylmethoxy)-2-naphthyl]propanamidomethyl]benzoate

Following the process described in example 1 (point F), starting from 2-(6-(2-quinolinylmethoxy)-2-naphthyl] propionic acid and methyl 4-aminomethylbenzoate, the title compound was prepared as a white solid with melting point 149.0-150.8°C (68% yield).
$^1$H N.M.R. (300 MHz, $CDCl_3$) δ ppm: 1.09 (d, 3H); 3.79 (s, 3H); 3.85 (q, 1H); 4.32 (s, 2H); 5.61 (s, 2H); 7.08 (m, 4H); 7.30 (m, 2H); 7.47 (m, 2H); 7.62 (m, 3H); 7.74 (t, 1H); 7.82 (d, 1H); 7.89 (d, 1H); 8.45 (d, 1H); 8.52 (d, 1H).

Example 12: 4-[2-[6-(2-Quinolinylmethoxy)-2-naphthyl]propanamidomethyl]benzoic acid

Following the process described in example 1 (point C), starting from methyl 4-[2-[6-(2-quinolinylmethoxy)-2-naphthyl]propanamidomethyl]benzoate, the title compound was prepared as a white solid with melting point 203.9-205.8°C (68% yield).
$^1$H N.M.R. (300 MHz, $CD_3OD$) δ ppm: 1.55 (d, 3H); 3.85 (q, 1H); 4.40 (q, 1H); 5.66 (s, 2H); 7.22 (d, 2H); 7.36 (dd, 1H); 7.41 (d, 1H); 7.47 (dd, 1H); 7.80 (m, 6H); 8.01 (m, 2H); 8.16 (d, 1H); 8.24 (d, 1H); 8.81 (d, 1H).

Example 13: methyl 3-[2-[6-(2-quinolinylmethoxy)-2-naphthyl]propanamidomethyl]benzoate

13A Methyl 3-cyanobenzoate

Following the process described in example 9 (point A), starting from 3-cyanobenzoic acid, the title compound was prepared as a yellowish oil (80% yield).
$^1$H N.M.R. (300 MHz, $CDCl_3$) δ ppm: 3.93 (s, 3H); 7.56 (t, 1H); 7.81 (dd, 1H); 8.23 (dd, 1H); 8.29 (d, 1H).

13B Methyl 3-aminomethylbenzoate

Following the process described in example 1 (point E), starting from methyl 3-cyanobenzoate and reacting for 4 h, the title compound was prepared as a semi-solid oil (92% yield).
$^1$H N.M.R. (300 MHz, $CD_3OD$) δ ppm: 3.88 (s, 3H); 3.91 (s, 2H); 7.49 (t, 1H); 7.51 (dd, 1H); 7.91 (dd, 1H); 7.99 (d, 1H).

13C Methyl 3-[2-[6-(2-quinolinylmethoxy)-2-naphthyl]propanamidomethyl]benzoate

Following the process described in example 1 (point F), starting from 2-[6-(2-quinolinylmethoxy)-2-naphthyl] propionic acid and methyl 3-aminomethylbenzoate, the title compound was prepared as a white solid with melting point 135.4-136.8°C (63% yield).
$^1$H N.M.R. (300 MHz, $CDCl_3$) δ ppm: 1.56 (d, 3H); 3.69 (q, 1H); 3.79 (s, 3H); 4.36 (d, 2H); 5.43 (s, 2H); 7.16 (d, 1H); 7.27 (m, 4H); 7.50 (t, 1H); 7.70 (m, 8H); 8.05 (d, 1H); 8.13 (d, 1H).

Example 14: 3-[2-[6-(2-quinolinylmethoxy)-2-naphthyl]propanamidomethyl]benzoic acid

Following the process described in example 1 (point C), starting from methyl 3-[2-[6-(2-quinolinylmethoxy)-2-naphthyl]propanamidomethyl]benzoate, the title compound was prepared as a white solid with melting point 203.5-204.7°C (77% yield).
$^1$H N.M.R. (300 MHz, $CDCl_3$) δ ppm: 1.43 (d, 3H); 3.80 (q, 1H); 4.27 (q, 2H); 7.25 (m, 3H); 7.42 (m, 2H); 7.62 (m, 1H);

7.75 (m, 5H); 8.00 (d, 1H); 8.05 (d, 1H); 8.32 (s, 1H); 8.42 (d, 1H); 8.56 (t, 1H).

Example 15: methyl 4-[2-[6-(2-quinolinylmethoxy)-2-naphthyl]propanamido]phenylacetate

15A Methyl 4-aminophenylacetate

Following the process described in example 9 (point A), starting from 4-aminophenylacetic acid, the title compound was prepared as a yellowish oil (73% yield).
$^1$H N.M.R. (300 MHz, $CDCl_3$) δ ppm: 3.50 (s, 2H); 3.65 (s, 3H); 3.68 (s, 2H); 6.59 (d, 2H); 7.04 (d, 2H).

15B Methyl 4-[2-[6-(2-quinolinylmethoxy)-2-naphthyl]propanamido]phenylacetate

Following the process described in example 1 (point F), starting from 2-(6-(2-quinolinylmethoxy)-2-naphthyl] propionic acid and methyl 4-aminophenylacetate, the title compound was prepared as a white solid with melting point 154.0-156.0°C (80% yield).
$^1$H N.M.R. (300 MHz, $CDCl_3$) δ ppm: 1.64 (d, 3H); 3.53 (s, 2H); 3.64 (s, 3H); 3.83 (q, 1H); 5.50 (s, 2H); 7.03 (m, 1H); 7.15 (d, 2H); 7.31 (m, 4H); 7.56 (t, 1H); 7.72 (m, 5H); 7.83 (d, 1H); 8.11 (d, 1H); 8.19 (d, 1H).

Example 16: 4-[2-[6-(2-quinolinylmethoxy]-2-naphthyl]propanamido]phenylacetic acid

Following the process described in example 1 (point C), starting from methyl 4-[2-[6-(2-quinolinylmethoxy)-2-naphthyl]propanamido]phenylacetate, the title compound was prepared as a white solid with melting point 141.0-143.2°C (77% yield).
$^1$H N.M.R. (300 MHz, $CDCl_3$-$CD_3OD$) δ ppm: 1.69 (d, 3H); 3.63 (s, 2H); 4.04 (q, 1H); 5.60 (s, 2H); 7.28 (d, 2H); 7.39 (d, 2H); 7.59 (d, 3H); 7.72-7.80 (m, 2H); 7.86-7.96 (m, 4H); 8.05 (d, 1H); 8.22 (d, 1H); 8.53 (d, 1H).

Example 17: methyl 3-[2-[6-(2-quinolinylmethoxy)-2-naphthyl]propanamido]phenylacetate

17A Methyl 3-aminophenylacetate

Following the process described in example 9 (point A), starting from 3-aminophenylacetic acid, the title compound was prepared as a yellowish oil (81% yield).
$^1$H N.M.R. (300 MHz, $CDCl_3$) δ ppm: 3.52 (s, 2H); 3.67 (s, 3H); 3.68 (s, 2H); 6.55 (m, 2H); 6.65 (d, 1H); 7.09 (t, 1H).

17B Methyl 3-[2-[6-(2-quinolinylmethoxy)-2-naphthyl]propanamido]phenylacetate

Following the process described in example 1 (point F), starting from 2-(6-(2-quinolinylmethoxy)-2-naphthyl] propionic acid and methyl 3-aminophenylacetate, the title compound was prepared as a white solid with melting point 141.8-142.8°C (75% yield).
$^1$H N.M.R. (300 MHz, $CDCl_3$) δ ppm: 1.65 (d, 3H); 3.54 (s, 2H); 3.64 (s, 3H); 3.82 (q, 1H); 5.51 (s, 2H); 6.96 (d, 1H); 7.03 (s, 1H); 7.35 (m, 6H); 7.56 (t, 1H); 7.75 (m, 6H); 8.12 (d, 1H).

Example 18: 3-[2-[6-(2-Quinolinylmethoxy)-2-naphthyl]propanamido]phenylacetic acid

Following the process described in example 1 (point C), starting from methyl 3-[2-[6-(2-quinolinylmethoxy)-2-naphthyl]propanamido]phenylacetate, the title compound was prepared as a white solid with melting point 200.9-202.9°C (61% yield).
$^1$H N.M.R. (300 MHz, $CDCl_3$-$CD_3OD$) δ ppm: 1.59 (d, 3H); 3.55 (s, 2H); 3.97 (q, 1H); 5.79 (s, 2H); 6.99 (d, 1H); 7.22 (t, 1H); 7.45 (m, 6H); 7.77 (d, 1H); 7.83 (m, 2H); 7.90 (m, 1H); 8.17 (m, 2H); 8.31 (dd, 1H); 8.37 (d, 1H); 9.1 (m, 1H).

Example 19: methyl 4-[4-[2-[6-[(7-chloro-2-quinolinyl)methoxy]-2-naphthyl]propanamido]phenyl]butanoate

19A 2-Bromomethyl-7-chloroquinoline

N-bromosuccinimide (3.3 g, 18.5 mmol) and some crystals of 2,2'-azobis(2-methylpropionitrile) (AIBN) were added to a solution of 7-chloro-2-methylquinoline (3 g, 16.9 mmol) in dry carbon tetrachloride (100 ml). The reaction mixture was refluxed for 4 h, then cooled at room temperature. The precipitate was filtered off and the filtrate was washed with a NaCl saturated solution (3x20 ml), dried and the solvent was evaporated off to obtain a crude which

was purified by flash chromatography through a silica gel column. Eluting with petroleum ether:chloroform, 3:2, 2.3 g of the title compound were obtained as a white solid with melting point 110-111°C (52% yield).
$^1$H N.M.R. (300 MHz, $CDCl_3$) δ ppm: 4.65 (s, 2H); 7.47 (dd, 1H); 7.53 (d, 1H); 7.71 (d, 1H); 8.03 (d, 1H); 8.11 (d, 1H).

19B Methyl 2-[6-(7-chloro-2-quinolinyl)methoxy-2-naphthyl]propionate

Following the process described in example 1 (point B), starting from methyl 2-(6-hydroxy-2-naphthyl)propionate and 2-bromomethyl-7-chloroquinoline, the title compound was prepared as a white solid with melting point 98-99°C (78% yield)
$^1$H N.M.R. (300 MHz, $CDCl_3$) δ ppm: 1.56 (d, 3H); 3.65 (s, 3H); 3.82 (q, 1H); 5.47 (s, 2H); 7.19 (d, 1H); 7.25 (s, 1H); 7.28 (dd, 1H); 7.37 (dd, 1H); 7.50 (dd, 1H); 7.70 (m, 4H); 8.10 (d, 1H); 8.14 (d, 1H).

19C 2-[6-(7-Chloro-2-quinolinyl)methoxy-2-naphthyl]propanoic acid

Following the process described in example 1 (point C), starting from methyl 2-[6-(7-chloro-2-quinolinyl)methoxy-2-naphthyl]propionate, the title compound was prepared (90% yield).
$^1$H N.M.R. (300 MHz, $CDCl_3$) δ ppm: 1.48 (d, 3H); 3.70 (q, 1H); 5.49 (d, 2H); 7.25 (m, 2H); 7.49 (dd, 1H); 7.58 (dd, 1H); 7.63 (d, 1H); 7.77 (m, 3H); 7.93 (d, 1H); 8.05 (d, 1H); 8.37 (d, 1H).

19D Methyl 4-[4-[2-[6-[(7-chloro-2-quinolinyl)methoxy]-2-naphthyl]propanamido]phenyl]butanoate

Following the process described in example 1 (point F), starting from 2-[6-(7-chloro-2-quinolinyl)methoxy-2-naphthyl]propanoic acid and methyl 4-(4-aminophenyl)butanoate, the title compound was prepared as a white solid with melting point 131.0-133.0°C (60% yield).
$^1$H N.M.R. (300 MHz, $CDCl_3$) δ ppm: 1.58 (d, 3H); 1.80 (q, 2H); 2.20 (t, 2H); 2.48 (t, 2H); 3.56 (s, 3H); 3.75 (q, 1H); 5.41 (s, 2H); 7.06 (m, 3H); 7.23 (dd, 1H); 7.30 (m, 3H); 7.41 (dd, 1H); 7.50 (dd, 1H); 7.73 (m, 4H); 8.10 (d, 1H); 8.16 (d, 1H).

Example 20: 4-[4-[2-[6-[(7-chloro-2-quinolinyl)-methoxy]-2-naphthyl]propanamido]phenyl]butanoic acid

Following the process described in example 1 (point C), starting from methyl 4-[4-[2-[6-[(7-chloro2-quinolinyl) methoxy]-2-naphthyl]propanamido]phenyl]butanoate, the title compound was prepared as a white solid with melting point 176.4-177.8°C (69% yield).
$^1$H N.M.R. (300 MHz, DMSO) δ ppm: 1.42 (d, 3H); 1.69 (q, 2H); 2.11 (t, 2H); 3.89 (q, 1H); 5.43 (s, 2H); 7.03 (d, 2H); 7.26 (dd, 1H); 7.37 (d, 1H); 7.45 (m, 3H); 7.61 (dd, 1H); 7.71 (m, 3H); 7.81 (d, 1H); 8.01 (d, 1H); 8.05 (s, 1H); 8.42 (d, 1H); 9.99 (s, 1H).

Example 21: N-[4-(1H-5-tetrazolyl)phenylmethyl]-6-(2-quinolinylmethoxy)-2-naphthaleneacetamide

21A 2-Acetyl-6-methoxynaphthalene

2-methoxynaphthalene (10 g, 63.3 mmol) followed by acetyl chloride (5.8 ml, 79.8 mmol) were added drop by drop to a solution of aluminium trichloride (10.9 g, 81.7 mmol) in nitrobenzene (50 ml) cooled at 0°C and under inert atmosphere. The reaction mixture was stirred at 0°C for 2 h and at room temperature for 18 h, then it was cooled to 0°C, poured onto ice (50 ml), added with concentrated HCl (20 ml) and chloroform (25 ml). The two phases were separated and the organic one was washed with water (3x10 ml), dried and the solvent was evaporated off, to obtain a crude which was purified by crystallization in methanol, thereby obtaining 7.9 g of the title compound as a white solid with melting point 107-109°C (63% yield).
$^1$H N.M.R. (300 MHz, $CDCl_3$) δ ppm: 2.69 (s, 3H); 3.93 (s, 3H); 7.17 (m, 2H); 7.75 (d, 1H); 7.83 (d, 1H); 7.99 (dd, 1H); 8.37 (s, 1H).

21B Methyl 6-methoxy-2-naphthaleneacetate

A mixture consisting of 2-acetyl-6-methoxynaphthalene (3 g, 15.0 mmol), sulfur (0.72 g, 22.5 mmol) and morpholine (2 ml) was refluxed for 18 h, then acetic acid (11 ml) and concentrated HCl (18 ml) were added and reflux was continued for a further 24 h. After that the mixture was evaporated to dryness, added with methanol (60 ml) and concentrated H2SO4 (10 ml) and refluxed for 18 h. Finally the mixture was evaporated to dryness, added with ethyl acetate, washed with a 5% $NaHCO_3$ saturated solution until the washing were neutral, dried and the solvent was evaporated

off, to obtain a crude, which was purified by flash chromatography through a silica gel column. Eluting with petroleum ether:chloroform, 4:1, 2.5 g of the title compound were obtained as a white solid with melting point 75.7-76.5°C (72% yield).
$^1$H N.M.R. (300 MHz, $CDCl_3$) δ ppm: 3.90 (s, 3H); 3.96 (s, 2H); 4.11 (s, 3H); 7.34 (m, 2H); 7.57 (dd, 1H); 7.85 (s, 1H); 7.88 (d, 1H); 7.90 (d, 1H).

21C Methyl 6-hydroxy-2-naphthaleneacetate

Following the process described in example 1 (point A), starting from methyl 6-methoxy-2-naphthaleneacetate, the title compound was prepared as a yellowish solid with melting point 84.8-85.8°C (72% yield)
$^1$H N.M.R. (300 MHz, $CDCl_3$) δ ppm: 3.74 (s, 3H); 3.77 (s, 2H); 6.16 (m, 1H); 7.03 (m, 2H); 7.32 (dd, 1H); 7.56 (m, 3H).

21D Methyl 6-(2-quinolinylmethoxy)-2-naphthaleneacetate

Following the process described in example 1 (point B), starting from methyl 6-hydroxy-2-naphthaleneacetate, the title compound was prepared as a white solid with melting point 106.3-107.9°C (82% yield)
$^1$H N.M.R. (300 MHz, $CDCL_3$) δ ppm: 3.72 (s, 3H); 3.78 (s, 2H); 5.64 (s, 2H); 7.26 (d, 1H); 7.33 (dd, 1H); 7.38 (dd, 1H); 7.59 (t, 1H); 7.74 (m, 5H); 7.86 (d, 3H); 8.15 (d, 1H); 8.22 (d, 1H).

21E 6-(2-Quinolinylmethoxy)-2-naphthaleneacetic acid

Following the process described in example 1 (point C), starting from methyl 6-(2-quinolinylmethoxy)-2-naphthaleneacetate, the title compound was prepared (84% yield).
$^1$H N.M.R. (300 MHz, DMSO) δ ppm: 3.75 (s, 2H); 5.60 (s, 2H); 7.20 (d, 1H); 7.31 (dd, 1H); 7.35 (dd, 1H); 7.60 (t, 1H); 7.68-7.83 (m, 6H); 8.20 (d, 1H); 8.25 (d, 1H).

21F N-[4-(1H-5-Tetrazolyl)phenylmethyl]-6-(2-quinolinylmethoxy)-2-naphthaleneacetamide

Following the process described in example 1 (point F), starting from 6-(2-quinolinylmethoxy)-2-naphthaleneacetic acid and 4-(1H-5-tetrazolyl)benzylamine hydrochloride, the title compound was prepared as a white solid with melting point 256-257°C (58% yield).
$^1$H N.M.R. (300 MHz, DMSO) δ ppm: 3.66 (s, 2H); 4.39 (d, 2H); 5.31 (s, 2H); 7.40 (m, 5H); 7.67 (t, 1H); 7.82 (m, 6H); 7.99 (m, 3H); 8.47 (d, 1H); 8.73 (t, 1H).

Example 22: methyl 4-[4-[[6-(2-quinolinylmethoxy)--2-naphthyl]carboxamido]phenyl]butanoate

22A 2-Cyano-6-hydroxynaphthalene

Following the process described in example 1 (point A), starting from 2-cyano-6-methoxynaphthalene, the title compound was prepared (91% yield).
$^1$H N.M.R. (300 MHz, $CD_3OD$) δ ppm: 7.17 (m, 2H); 7.51 (dd, 1H); 7.74 (d, 1H); 7.82 (d, 1H); 8.22 (s, 1H).

22B 2-Cyano-6-(2-quinolinylmethoxy)naphthalene

Following the process described in example 1 (point B), starting from 2-cyano-6-hydroxynaphthalene, the title compound was prepared as a white solid with melting point 155.0-155.8°C (70% yield).
$^1$H N.M.R. (300 MHz, $CDCl_3$) δ ppm: 5.30 (s, 2H); 7.34 (d, 1H); 7.46 (dd, 1H); 7.60 (m, 2H); 7.80 (m, 5H); 8.18 (m, 2H); 8.27 (d, 1H).

22C 6-(2-Quinolinylmethoxy)-2-naphthoic acid

A solution of 35% sodium hydroxide (35 ml) was added to a solution of 2-cyano-6-(2-quinolinylmethoxy)naphthalene (0.779, 2.5 mmol) in ethanol (130 ml) and refluxed for 24 h. After that the reaction was cooled at room temperature, added 1M HCl to pH 5 and left to stand at 5-10°C for 24 h. After this time, the resulting precipitate was filtered, washed with cold ethanol and dried over phosphorous pentoxide, thereby obtaining 0.796 g of the title compound as a white solid with melting point 239-240°C (96% yield).
$^1$H N.M.R. (300 MHz, DMSO) δ ppm: 5.53 (s, 1H); 7.39 (dd, 1H); 7.54 (s, 1H); 7.62 (t, 2H); 7.76 (m, 3H); 8.00 (m, 4H); 8.42 (d, 1H); 8.53 (s, 1H).

22D Methyl 4-[4-[[6-(2-quinolinylmethoxy)-2-naphthyl]carboxamido]phenyl]butanoate

Following the process described in example 1 (point F), starting from 6-(2-quinolinylmethoxy)-2-naphthoic acid and methyl 4-(4-aminophenyl)butanoate, the title compound was prepared (70% yield).
$^1$H N.M.R. (300 MHz, DMSO) δ ppm: 1.75 (q, 2H); 2.17 (t, 2H); 2.53 (t, 2H); 3.60 (s, 3H); 5.50 (s, 2H); 7.13 (d, 1H); 7.38 (dd, 1H); 7.53 (d, 1H); 7.59 (dt, 1H); 7.73 (m, 4H); 7.86 (d, 1H); 7.95 (m, 4H); 8.40 (d, 1H); 8.47 (s, 1H); 10.25 (s, 1H).

Example 23: 4-[4-[[6-(2-quinolinylmethoxy)-2-naphthyl]carboxamido]phenyl]butanoic acid

Following the process described in example 1 (point C), starting from methyl 4-[4-[[6-(2-quinolinylmethoxy)-2-naphthyl]carboxamido]phenyl]butanoate, the title compound was prepared as a white solid with melting point 231.9-232.5°C (78% yield).
$^1$H N.M.R. (300 MHz, DMSO) δ ppm: 1.75 (q, 2H); 2.17 (t, 2H); 2.53 (t, 2H); 5.50 (s, 2H); 7.13 (d, 1H); 7.38 (dd, 1H); 7.53 (d, 1H); 7.59 (dt, 1H); 7.73 (m, 4H); 7.86 (d, 1H); 7.95 (m, 4H); 8.40 (d, 1H); 8.47 (s, 1H); 10.25 (s, 1H).

Example 24: N-[3-(1H-5-tetrazolyl)phenylmethyl]-6-(2-quinolinylmethoxy)-2-naphthalenecarboxamide

Following the process described in example 1 (point F), starting from 6-(2-quinolinylmethoxy)-2-naphthoic acid and 3-(1H-5-tetrazolyl)benzylamine, the title compound was prepared as a white solid with melting point 234.5-235.0°C (68% yield).
$^1$H N.M.R. (300 MHz, DMSO) δ ppm: 4.63 (d, 2H); 5.53 (s, 2H); 7.41 (dd, 1H); 7.56 (m, 3H); 7.63 (t, 1H); 7.75 (d, 1H); 7.81 (dt, 1H); 7.92 (m, 3H); 8.01 (m, 4H); 8.45 (d, 1H); 8.48 (s, 1H); 9.26 (t, 1H).

Example 25: methyl 4-[4-[[7-(2-quinolinylmethoxy)-2-naphthyl]carboxamido]phenyl]butanoate

25A 2-t-Butyldimethylsilyloxy-7-hydroxynaphthalene

t-butyldimethylsilyl chloride (4.71 g, 31.2 mmol) was added to a solution of 2,7-dihydroxynaphthalene (5 g, 0.031 mmol) and imidazole (1.9 g, 0.028 mmol) in dry DMF (30 ml), cooled at 0°C and under inert atmosphere. The reaction mixture was stirred for 2.5 h. After that ethyl ether (100 ml) was added to the reaction mixture, to give a precipitate which was filtered off. The filtrate was washed with a NaCl saturated solution (3x20 ml), dried and the solvent was evaporated off, to obtain a crude which was purified by flash chromatography through a silica gel column, eluting with petroleum ether:ethyl ether to obtain 6.0 g of the title compound as a white solid with melting point 104.8-105.8°C (70% yield).
$^1$H N.M.R. (300 MHz, $CDCl_3$) δ ppm: 0.30 (s, 6H); 1.07 (s, 9H); 5.86 (m, 1H); 7.00 (m, 4H); 7.69 (d, 1H); 7.70 (d, 1H).

25B: 2-t-Butyldimethylsilyloxy-7-trifluoromethylsulfoxynaphthalene

Pyridine (1.66 ml) and trifluoromethanesulfonic anhydride (4.32 g, 15.2 mmol) was added to a solution of 2-t-butyldimethylsilyloxy-7-hydroxynaphthalene (3.5 g, 12.7 mmol) in methylene chloride (15 ml), cooled at 0°C and under inert atmosphere. The reaction mixture was stirred at this temperature for 1 h, then diluted with ethyl ether (100 ml), washed in succession with 0.01M HCl, a $NaHCO_3$ saturated solution and a NaCl saturated solution, dried and the solvent was evaporated off, to obtain 5 g of the title compound as an orange oil (97% yield).
$^1$H N.M.R. (300 MHz, $CDCl_3$) δ ppm: 7.24 (m, 3H); 7.67 (s, 1H); 7.81 (d, 1H); 7.87 (d, 1H).

25C Methyl 7-t-butyldimethylsilyloxy-2-naphthalenecarboxylate

Triethylamine (3.2 ml, 23.3 mmol), dimethyl sulfoxide (31 ml) Pd(OAc)2 (0.069 g, 0.31 mmol) and 1,3-bis (diphenylphosphino)propane (0.128 g, 0.31 mmol) were added to a solution of 2-t-butyldimethylsilyloxy-7-trifluoromethylsulfoxynaphthalene (3.6 g, 10.6 mmol) in absolute methanol (20 ml), . The mixture was subjected to a carbon monoxide stream for 4 min, heated to a 75°C for 3 h under carbon monoxide atmosphere, cooled at room temperature, filtered through celite and methanol was evaporated off. The resulting solution was diluted with ethyl ether and washed in succession with water, 5% HCl, a 5% $NaHCO_3$ solution and a NaCl saturated solution, dried and the solvent was evaporated off, to obtain a crude which was purified by flash chromatography through a silica gel column. Eluting with petroleum ether:ethyl ether 98:2, 2.0 g of the title compound were prepared as a yellowish oil (60% yield).
$^1$H N.M.R. (300 MHz, $CDCl_3$) δ ppm: 0.27 (s, 6H); 1.04 (s, 9H); 3.95 (s, 3H); 7.20 (dd, 1H); 7.32 (d, 1H); 7.84 (m, 3H); 8.44 (s, 1H).

25D Methyl 7-hydroxy-2-naphthalenecarboxylate

$K_2CO_3$ (1.5 g, 12.5 mmol) was added to a solution of methyl 7-t-butyldimethylsilyloxy-2-naphthalenecarboxylate (1.30 g, 4.17 mmol) in tetrahydrofuran (40 ml) and methanol (40 ml) and stirred at room temperature for 4.5 h under nitrogen atmosphere. A $NH_4Cl$ saturated solution and ethyl ether were added thereto, the phases were separated and the aqueous one was extracted with ethyl ether. The combined organic extracts were washed with water, dried and the solvent was evaporated off, to obtain a crude which was purified by flash chromatography through a silica gel column. Eluting with petroleum ether:ethyl acetate, 4:1, 0.96 g of the title compound were obtained (98% yield).
$^1$H N.M.R. (300 MHz, $CD_3OD$) δ ppm: 3.96 (s, 3H); 7.22 (m, 2H); 7.80 (m, 3H); 8.39 (s, 1H).

25E Methyl 7-(2-quinolinylmethoxy)-2-naphthalenecarboxylate

Following the process described in example 1 (point B), starting from methyl 7-hydroxy-2-naphthalenecarboxylate, the title compound was prepared (97% yield).
$^1$H N.M.R. (300 MHz, $CDCl_3$) δ ppm: 3.98 (s, 3H); 5.55 (s, 2H); 7.36 (d, 1H); 7.43 (dd, 1H); 7.59 (dt, 1H); 7.81 (m, 5H); 7.95 (dd, 1H); 8.16 (d, 1H); 8.23 (d, 1H); 8.47 (s, 1H).

25F 7-(2-Quinolinylmethoxy)-2-naphthoic acid

Following the process described in example 1 (point C), starting from methyl 7-(2-quinolinylmethoxy)-2-naphthalenecarboxylate, the title compound was prepared as a white solid with melting point 227.8-228.8°C (83% yield).
$^1$H N.M.R. (300 MHz, $CD_3OD$-$CDCl_3$) δ ppm: 5.52 (s, 2H); 7.42 (m, 2H); 7.62 (t, 1H); 7.76-7.95 (complex signal, 6H); 8.11 (d, 1H); 8.33 (d, 1H); 8.98 (s, 1H).

25G Methyl 4-[4-[[7-(2-quinolinylmethoxy)-2-naphthyl]carboxamido]phenyl]butanoate

Following the process described in example 1 (point F), starting from 7-(2-quinolinylmethoxy)-2-naphthoic acid and methyl 4-(4-aminophenyl)butanoate, the title compound was prepared as a white solid with melting point 166.4-167.9°C (64% yield).
$^1$H N.M.R. (300 MHz, $CDCl_3$) δ ppm: 1.84 (q, 2H); 2.23 (t, 2H); 2.52 (t, 2H); 7.05 (d, 2H); 7.12 (d, 1H); 7.26 (dd, 1H); 7.60 (m, 8H); 8.06 (m, 4H).

Example 26: 4-[4-[[7-(2-quinolinylmethoxy)-2-naphthyl]carboxamido]phenyl]butanoic acid

Following the process described in example 1 (point C), starting from methyl 4-[4-[[7-(2-quinolinylmethoxy)-2-naphthyl]carboxamido]phenyl]butanoate, the title compound was prepared as a white solid with melting point 300-302°C (80% yield).
$^1$H N.M.R. (300 MHz, DMSO) δ ppm: 1.72 (q, 2H); 1.92 (t, 2H); 2.50 (m, 2H); 5.54 (s, 2H); 7.15 (d, 2H); 7.45 (dd, 1H); 7.59 (d, 1H); 7.70 (m, 6H); 7.90 (m, 4H); 8.33 (s, 1H); 8.44 (m, 2H).

Example 27: N-[4-(1H-5-tetrazolyl)phenylmethyl]-7-(2-quinolinylmethoxy)-2-naphthalenecarboxamide

Following the process described in example 1 (point F), starting from 7-(2-quinolinylmethoxy)-2-naphthoic acid and 4-(1H-5-tetrazolyl)benzylamine hydrochloride, the title compound was prepared as a white solid with melting point 224.4-225.9°C (87% yield).
$^1$H N.M.R. (300 MHz, DMSO) δ ppm: 4.58 (d, 2H); 5.53 (s, 2H); 7.44 (dd, 1H); 7.54 (m, 3H); 7.62 (t, 1H); 7.74 (d, 1H); 7.81 (m, 2H); 7.99 (m, 6H); 8.36 (s, 1H); 8.43 (d, 1H); 9.24 (t, 1H).

Example 28: ethyl 4-[2-[[7-(2-quinolinylmethoxy)-2-naphthyl]carboxamido]ethyl]benzoate

28A Methyl 4-cyanomethylbenzoate

Methyl 4-chloromethylbenzoate (8 g, 43.3 mmol) dissolved in ethanol (6 ml) was added to a solution of sodium cyanide (2.5 g, 51.0 mmol) in water (3 ml) and left at 100°C for 3 h. The reaction mixture was cooled at room temperature, added with ethyl ether (30 ml) and a NaCl saturated solution (10 ml). The two phases were separated and the aqueous one was extracted with ethyl ether (3x25 ml). The ether extracts were dried and the solvent was evaporated off, to obtain a crude which was purified by flash chromatography through a silica gel column. Eluting with petroleum ether: ethyl acetate, 3:2, 6.1 g of the title compound were prepared as a yellowish oil (80% yield).

$^1H$ N.M.R. (300 MHz, $CDCl_3$) δ ppm: 3.85 (s, 2H); 3.95 (s, 2H); 7.44 (d, 2H); 8.07 (d, 2H).

28B Methyl 4-(2-aminoethyl)benzoate

Following the process described in example 1 (point E), starting from methyl 4-cyanomethylbenzoate, the title compound was prepared as a semi-solid oil (90% yield).
$^1H$ N.M.R. (300 MHz, $CD_3OD$) δ ppm: 3.07 (t, 2H); 3.32 (t, 2H); 3.92 (s, 3H); 7.44 (d, 2H); 8.03 (d, 2H).

28C Ethyl 4-[2-[[7-(2-quinolinylmethoxy)-2-naphthyl]carboxamido]ethyl]benzoate

Following the process described in example 1 (point F), starting from 7-(2-quinolinylmethoxy)-2-naphthoic acid and ethyl 4-(2-aminoethyl)benzoate, the title compound was prepared as a white solid with melting point 212.2-213.4°C (83% yield).
$^1H$ N.M.R. (300 MHz, $CD_3OD$-$CDCl_3$) δ ppm: 3.02 (t, 2H); 3.74 (m, 2H); 3.90 (s, 3H); 5.49 (s, 2H); 6.97 (t, 1H); 7.34 (m, 3H); 7.61 (m, 2H); 7.71-7.86 (complex signal, 5H); 7.98 (d, 2H); 8.00 (m, 2H); 8.23 (d, 1H).

Example 29: 4-[2-[[7-(2-quinolinylmethoxy)-2-naphthyl]carboxamido]ethyl]benzoic acid

Following the process described in example 1 (point C), starting from ethyl 4-[2-[[7-(2-quinolinylmethoxy)-2-naphthyl]carboxamido]ethyl]benzoate, the title compound was prepared as a white solid with melting point 228.0-229.0°C (63% yield).
$^1H$ N.M.R. (300 MHz, DMSO) δ ppm: 2.92 (t, 2H); 3.53 (t, 2H); 5.53 (s, 2H); 7.32 (d, 2H); 7.42 (dd, 1H); 7.51 (d, 1H); 7.63 (dt, 1H); 7.72-7.94 (complex signal, 7H); 8.01 (d, 1H); 8.06 (d, 1H); 8.25 (s, 1H); 8.44 (d, 1H); 8.70 (t, 1H).

Example 30: N-[4-(1H-5-tetrazolyl)phenylethyl]-7-(2-quinolinylmethoxy)-2-naphthalenecarboxamide

30A Methyl 4-[2-(t-butoxycarbonylamino)ethyl]benzoate

1M NaOH solution (15.4 ml) and di-t-butyl dicarbonate (2.27 g, 10.4 mmol) were added to a solution of methyl 4-(2-aminoethyl)benzoate (1.5 g, 6.96 mmol) in dioxane (30 ml) and water (15 ml) at 0°C. The reaction mixture was stirred at room temperature for 18 h, keeping pH at 9-10 throughout the reaction by means of several additions of 1M NaOH. Dioxane was evaporated off and the aqueous residue was acidified to pH 3 with 1M HCl, extracted with ethyl acetate (3x50 ml), dried and the solvent was evaporated off to obtain 1.6 g of the title compound (92% yield).
$^1H$ N.M.R. (300 MHz, $CDCl_3$) δ ppm: 1.42 (s, 9H); 2.83 (t, 2H); 3.38 (q, 2H); 3.88 (s, 3H); 7.27 (d, 2H); 7.98 (d, 2H).

30B 4-[2-(t-Butoxycarbonylamino)ethyl]benzoic acid

1M potassium hydroxide (34.1 ml) was added to a solution of methyl 4-[2-(t-butoxycarbonylamino)ethyl]benzoate (1.7 g, 6.82 mmol) in ethanol (380 ml) and stirred under reflux for 30 min. After that ethanol was removed, and the resulting solid residue was redissolved in water (35 ml), adjusting to pH 4-5 with 10% acetic acid, thereby obtaining a precipitate which was separated by filtration, washed with ethyl ether and dried over phosphorous pentoxide, to obtain 1.3 g of the title compound (91% yield).
$^1H$ N.M.R. (300 MHz, $CD_3OD$) δ ppm: 1.41 (s, 9H); 2.82 (t, 2H); 3.30 (m, 2H); 7.32 (d, 2H); 7.92 (d, 2H).

30C 4-[2-(t-Butoxycarbonylamino)ethyl]benzamide

Triethylamine (1.7 ml, 12.34 mmol) and ethyl chloroformate (0.64 ml, 6.79 mmol) were added to a solution of 4-[2-(t-butoxycarbonylaminoethyl]benzoic acid (1.45 g, 6.17 mmol) in dry THF (100 ml). The reaction mixture was stirred at room temperature for 30 min, then subjected to an ammonia stream for 30 min., evaporated to dryness and treated with chloroform to remove the ethyl chloroformate excess, thus obtaining 1.2 of the title compound (83% yield).
$^1H$ N.M.R. (300 MHz, $CD_3OD$) δ ppm: 1.40 (s, 9H); 2.81 (t, 2H); 3.32 (m, 2H); 7.32 (d, 2H); 7.92 (d, 2H).

30D 4-[2-(t-Butoxycarbonylamino)ethyl]benzonitrile

A solution of 4-[2-(t-butoxycarbonylamino)ethyl]benzamide (0.5 g, 2.13 mmol) in 2 ml of DMF was added to a solution of phosphorous oxychloride (1 ml, 2.13 mmol) in dry DMF (16 ml), kept at 0°C and under inert atmosphere for 30 min. The reaction mixture was stirred at room temperature for 24 h, then poured onto ice and extracted with ethyl acetate (4x25 ml), dried and the solvent was evaporated off, to obtain 0.35 g of the title compound (77% yield).

$^1$H N.M.R. (300 MHz, $CDCl_3$) δ ppm: 1.53 (s, 9H); 2.93 (t, 2H); 3.86 (t, 2H); 7.34 (d, 2H); 7.62 (d, 2H); 9.17 (s, 1H).

30E 5-[4-(2-t-Butoxycarbonylamino)phenyl]-1H-tetrazol

Following the process described in example 1 (point D), starting from 4-[2-(t-butoxycarbonylamino)ethyl]benzonitrile, the title compound was prepared (72% yield).
$^1$H N.M.R. (300 MHz, $CD_3OD$) δ ppm: 1.37 (s, 9H); 3.30 (t, 2H); 3.50 (t, 2H); 7.39 (d, 2H); 7.88 (d, 2H).

30F 2-[4-(1H-5-Tetrazolyl)phenyl]ethylamine

Trifluoroacetic acid (0.37 ml, 4.8 mmol) was added to a solution of 5-[4-(2-t-butoxycarbonylamino)phenyl]-1H-tetrazole (0.312 g, 1.20 mmol) in dry methylene chloride (4 ml) and stirred at room temperature for 18 h. After that the reaction mixture was evaporated to dryness to obtain 0.358 g of the title compound as the trifluoroacetic acid salt (99% yield).
$^1$H N.M.R. (300 MHz, $CD_3OD$) δ ppm: 3.09 (t, 2H); 3.28 (t, 2H); 7.45 (d, 2H); 7.94 (d, 2H).

30G N-[4-(1H-5-Tetrazolyl)phenylethyl]-7-(2-quinolinylmethoxy)-2-naphthalenecarboxamide

Following the process described in example 1 (point F), starting from 7-(2-quinolinylmethoxy)-2-naphthoic acid and 2-[4-(1H-5-tetrazolyl)phenyl]ethylamine, the title compound was prepared as a white solid with melting point 187.4-189.1°C (67% yield).
$^1$H N.M.R. (300 MHz, DMSO) δ ppm: 2.97 (t, 2H); 3.55 (m, 2H); 5.55 (s, 2H); 7.41-7.51 (complex signal, 4H); 7.63 (t, 1H); 7.78 (m, 3H); 7.89-8.08 (complex signal, 6H); 8.27 (s, 1H); 8.43 (s, 1H); 8.72 (t, 1H).

Example 31: ethyl 4-[4-[[6-(2-quinolinylmethoxy)-2-naphthyl]methylaminocarbonyl]phenyl]butanoate

31A 2-Aminomethyl-6-methoxynaphthalene

Following the process described in example 1 (point E), starting from 2-cyano-6-methoxynaphthalene, the title compound was prepared (90% yield).
$^1$H N.M.R. (300 MHz, $CD_3OD$) δ ppm: 3.92 (s, 3H); 4.25 (s, 2H); 7.19 (dd, 1H); 7.28 (d, 1H); 7.50 (dd, 1H); 7.79-7.88 (m, 3H).

31B 2-Acetylaminomethyl-6-methoxynaphthalene

Triethylamine (2.9 ml, 20.5 mmol) and acetic anhydride (0.73 ml, 7.69 mmol) were added to a solution of 2-aminomethyl-6-methoxynaphthalene (1.23 g, 6.40 mmol) in chloroform (200 ml), cooled at -30°C. The reaction mixture was left at this temperature for 2 h, then cooled at room temperature and added with water (50 ml), the two phases were separated and the organic one was washed with a 0.2M HCl solution, dried and the solvent was evaporated off, to obtain the title compound as a yellowish solid with melting point 163-165°C (86% yield).
$^1$H N.M.R. (300 MHz, $CDCl_3$) δ ppm: 2.03 (s, 3H); 3.90 (s, 3H); 4.53 (d, 2H); 5.95 (s, 1H); 7.13 (m, 2H); 7.35 (dd, 1H); 7.62 (s, 1H); 7.68 (m, 2H).

31C 6-Acetylaminomethyl-2-naphthol

Following the process described in example 1 (point A), starting from 2-acetylaminomethyl-6-methoxynaphthalene, the title compound was prepared as a white solid with melting point 219-222°C (93% yield).
$^1$H N.M.R. (300 MHz, $CD_3OD$) δ ppm: 2.09 (s, 3H); 4.50 (s, 2H); 7.06 (m, 2H); 7.32 (dd, 1H); 7.65 (m, 3H).

31D 2-Acetylaminomethyl-6-(2-quinolinylmethoxy)naphthalene

Following the process described in example 1 (point B), starting from 6-acetylaminomethyl-2-naphthol, the title compound was prepared as a semi-solid oil (65% yield).
$^1$H N.M.R. (300 MHz, $CDCl_3$) δ ppm: 1.93 (s, 3H); 4.49 (d, 2H); 5.44 (s, 2H); 5.85 (s, 1H); 7.25 (m, 3H); 7.51 (t, 1H); 7.67 (m, 5H); 7.78 (d, 1H); 8.07 (d, 1H); 8.15 (d, 1H).

31E 2-Aminomethyl-6-(2-quinolinylmethoxy)naphthalene

6M HC1 (2 ml) was added to a solution of 2-acetylaminomethyl-6-(2-quinolinylmethoxy)naphthalene (0.100 g, 0.281 mmol) in dioxane (10 ml) and refluxed for 18 h. After that the reaction mixture was cooled at room temperature, diluted with water (10 ml), 1M NaOH was added to basic pH and extracted with ethyl acetate. The organic extracts were dried and the solvent was evaporated off, to obtain 0.064 g of the title compound (73% yield).
$^1$H N.M.R. (300 MHz, $CD_3OD$) δ ppm: 3.89 (s, 2H); 5.34 (s, 2H); 7.25 (m, 2H); 7.36 (dd, 1H); 7.54 (t, 1H); 7.70 (m, 5H); 7.84 (d, 1H); 8.02 (d, 1H); 8.24 (d, 1H).

31F Ethyl 4-(4-formylphenyl)butanoate

Hexamethylenetetramine (1.6 g, 11.5 mmol) was added to a solution of ethyl 4-phenylbutanoate (2 g, 10.4 mmol) in trifluoroacetic acid (10 ml) and left at 80°C for 18 h. After that the reaction mixture was evaporated to dryness, added with a $NaHCO_3$ saturated solution (40 ml) and extracted with ethyl ether (4x50 ml). The ether extracts were dried and the solvent was evaporated off, to obtain a crude which was purified by flash chromatography through a silica gel column. Eluting with hexane:ethyl acetate, 9:1, 1.3 g of the title compound was prepared as a colourless oil (57% yield).
$^1$H N.M.R. (300 MHz, $CDCl_3$) δ ppm: 1.23 (t, 3H); 1.96 (m, 2H); 2.31 (t, 2H); 2.71 (t, 2H); 4.11 (q, 2H); 7.32 (d, 2H); 7.78 (d, 2H); 9.95 (s, 1H).

31G 4-(3-Ethoxycarbonylpropyl)benzoic acid

1 ml of Jones's reagent was added at 0°C to a solution of ethyl 4-(4-formylphenyl)butanoate (1.16 g, 5.29 mmol) in acetone (7 ml). The reaction mixture was stirred at room temperature for 18 h, then added with isopropanol (1 ml) and extracted with ethyl ether. The organic phase was dried and the solvent was evaporated off, to obtain 1.05 g of the title compound as a colourless oil (84% yield).
$^1$H N.M.R. (300 MHz, $CDCl_3$) δ ppm: 1.09 (t, 3H); 1.81 (m, 2H); 2.17 (t, 2H); 2.55 (t, 2H); 3.97 (q, 2H); 7.11 (d, 2H); 7.87 (d, 2H); 9.11 (m, 1H).

31H Ethyl 4-[4-[[6-(2-quinolinylmethoxy)-2-naphthyl]methylaminocarbonyl]phenyl]butanoate

Following the process described in example 1 (point F), starting from 2-aminomethyl-6-(2-quinolinylmethoxy) naphthalene and 4-(3-ethoxycarbonylpropyl)benzoic acid, the title compound was prepared (61% yield).
$^1$H N.M.R. (300 MHz, $CDCl_3$) δ ppm: 1.26 (t, 3H); 1.95 (m, 2H); 2.30 (t, 2H); 2.65 (t, 2H); 4.10 (q, 2H); 4.70 (d, 2H); 5.40 (s, 2H); 7.25 (m, 5H); 7.70 (m, 9H); 8.15 (t, 2H).

Example 32: 4-[4-[[6-(2-quinolinylmethoxy)-2-naphthyl]methylaminocarbonyl]phenyl]butanoic acid

Following the process described in example 1 (point C), starting from ethyl 4-[4-[[6-(2-quinolinylmethoxy)-2-naphthyl]methylaminocarbonyl]phenyl]butanoate, the title compound was prepared as a white solid with melting point 185.9-187.8 (80% yield).
$^1$H N.M.R. (300 MHz, $CDCl_3$) δ ppm: 1.81 (t, 2H); 2.22 (t, 2H); 2.64 (t, 2H); 4.60 (d, 2H); 5.48 (m, 5H); 7.40 (m, 5H); 7.75 (m, 8H); 8.03 (m, 2H); 8.42 (d, 1H); 9.05 (t, 1H).

Example 33: N-[4-(1*H*-5-tetrazolyl)phenylpropyl]-7-(2-quinolinylmethoxy)-2-naphthalenecarboxamide

33A 3-(4-Bromophenyl)propan-1-ol

A solution of 4-bromocinnamic acid (5.0 g, 22 mmol) in 20 ml of dry ethyl ether was added to a suspension of aluminium lithium hydride (2.49 g, 66 mmol) in dry ethyl ether (130 ml) under inert atmosphere. The reaction mixture was stirred at room temperature for 2 hours, then a NaCl saturated solution in water (80 ml) was slowly added, the two phases were separated and the aqueous one was extracted with ethyl acetate (3x50 ml). The organic extracts were dried and the solvent was evaporated off to obtain 3.60 g of the title compound as a yellowish oil (76% yield).
$^1$H N.M.R. (300 MHz, $CDCl_3$) δ ppm: 1.85 (m, 2H); 2.66 (t, 2H); 3.65 (t, 2H); 7.06 (d, 2H); 7.39 (d, 2H).

33B 3-(4-Cyanophenyl)propan-1-ol

A mixture of 3-(4-bromophenyl)propan-1-ol (2.0 g, 9.3 mmol), copper (I) cyanide (1.49 g, 16.7 mmol) and N-methylpyrrolidinone (13 ml) was stirred at 200°C for 2.5 hours. After that the reaction mixture was cooled at room temperature, poured onto a solution of diethylamine (30 g) and water (80 ml) and extracted with ethyl acetate (3x40 ml). The combined organic phases were dried and volatiles were removed, to obtain an oil from which N-methylpyrrolidinone was removed by distillation under high vacuum (0.5 torr, 85°C), thereby obtaining 0.78 g of the title compound (52% yield).
$^1$H N.M.R. (300 MHz, $CDCL_3$) δ ppm: 1.85 (m, 2H); 2.42 (s broad, 1H); 2.76 (t, 2H); 3.64 (t, 2H); 7.29 (d, 2H); 7.53 (d, 2H).

33C 3-(4-Cyanophenyl)propyl methanesulfonate

Triethylamine (0.54 ml, 4.03 mmol) and methanesulfonyl chloride (0.30 ml, 4.03 mmol) were added to a solution of 3-(4-cyanophenyl)propan-1-ol (0.50 g, 3.10 mmol) in dry methylene chloride (15 ml), cooled at 0°C and under inert atmosphere. The reaction mixture was stirred at 0°C for 2 hours, after that was diluted with methylene chloride (50 ml), washed in succession with 0.05M HCl, with a NaCl saturated solution, dried and the solvent was evaporated off. 0.675 g of the title compound were obtained as a semi-solid oil (94% yield).
$^1$H N.M.R. (300 MHz, $CDCl_3$) δ ppm: 1.89 (m, 2H); 2.63 (t, 2H); 2.85 (s, 3H); 4.05 (t, 2H); 7.14 (d, 2H); 7.38 (d, 2H).

33D 4-(1H-5-Tetrazolyl)azidopropylbenzene

Following the process described in example 1 (point D), starting from 3-(4-cyanophenyl)propyl methanesulfonate, the title compound was prepared (70% yield).
$^1$H N.M.R. (300 MHz, $CDCl_3$) δ ppm: 1.79 (m, 2H); 2.63 (t, 2H); 3.18 (t, 2H); 7.21 (d, 2H); 7.98 (d, 2H).

33E 3-[4-(1H-5-Tetrazolyl)phenyl]propylamine hydrochloride

Following the process described in example 1 (point E), starting from 4-(1H-5-tetrazolyl)azidopropylbenzene, the title compound was prepared as a white solid (crystallized from methanol) which decomposes at 251°C (87% yield).
$^1$H N.M.R. (300 MHz, $CD_3OD$) δ ppm: 2.03 (m, 2H); 2.83 (t, 2H); 3.00 (t, 2H); 7.48 (d, 2H); 7.97 (d, 2H).

33F N-[4-(1H-5-Tetrazolyl)phenylpropyl]-7-(2-quinolinylmethoxy)-2-naphthalenecarboxamide

Following the process described in example 1 (point F), starting from 7-(2-quinolinylmethoxy)-2-naphthoic acid and 3-[4-(1H-5-tetrazolyl)phenyl]propylamine, the title compound was prepared as a white solid with melting point 218.0-219.8°C (65% yield).
$^1$H N.M.R. (300 MHz, $CD_3OD$) δ ppm: 1.91 (m, 2H); 2.73 (t, 2H); 3.33 (t, 2H); 5.52 (s, 2H); 7.42 (dd, 1H), 7.45-7.54 (complex signal, 3H); 7.63 (t, 1H); 7.72-7.84 (complex signal, 3H); 7.88-8.00 (complex signal, 4H); 8.03 (dd, 1H); 8.25 (d, 1H); 8.28 (s, 1H); 8.44 (d, 1H); 8.63 (t broad, 1H).

Biological activity tests

The antagonistic activity on $LTD_4$ of the compounds of the present invention is determined by means of an inhibition test of the [$^3$H]-$LTD_4$ receptor binding in guinea-pig lung membranes, and a test of inhibition of $LTD_4$-induced contractions in the mienteric plexus of guinea-pig isolated ileum.

[$^3$H]-$LTD_4$ receptor binding inhibition test in quinea-pig lung membranes

Guinea pig lung membranes, containing the $LTD_4$ receptors, are purified following the method described by Mong et. al. (Mong et al., Prostaglandins, 28, 805 (1984)). These purified membranes (150 μg/ml) are added to an incubation mixture containing 10 mM of PIPES buffer (piperazin-N,N'-bis(2-ethanesulfonic acid) (pH 7.4), 10 mM of $CaCl_2$, 10 mM of 5 $MgCl_2$, 2 mM of cysteine, 2 mM of glycine, 0.5 nM of [$^3$H]-$LTD_4$ (4700-6400 GBq/mmol) and different concentrations of the product under test in a final volume of 310 μl. The reaction mixture is incubated for 30 minutes at 25°C.
The radioligand bound to the membranes is separated from the free one by dilution with 4 ml washing buffer (10 mM Tris-HCl (pH 7.4) and 100 mM NaCl) at 0°C and filtration with Whatman GF/B filters, by means of a Brandel Cell Harvester. The filters are washed 4 times with a total volume of 16 ml of washing buffer at 0°C. The radioactivity present in the filters is determined by liquid scintillation.
The specific binding is defined as the difference between the total binding of [$^3$H]-$LTD_4$ and the nonspecific binding determined in the presence of 1 μM $LTD_4$. The data obtained in the competition tests are analyzed by a computational program, which determines the inhibition constant of each compound ($K_i$) by means of the Cheng-Prusoff

equation (Cheng et al., Biochem. Pharmacol., 22, 3094 (1973)).

$$K_i = IC_{50} / (1 + [L] / K_d)$$

wherein $IC_{50}$ is the concentration of compound which desplaces a 50% of the bound radioligand, [L] is the concentration of $[^3H]LTD_4$ free in the test and $K_d$ is the dissociation constant of the $LTD_4$ obtained in an independent way by means of Scatchard analysis.

Table 1 shows some of the activity values found for the compounds of the present invention.

Inhibition test of the contractions induced by $LTD_4$ in the mienteric plexus of quinea-pig isolated ileum.

The antagonistic activity of the compounds of the present invention in the isolated organ was evaluated as its ability to inhibit the contraction caused by $LTD_4$ in the mienteric plexus of the ileum of Dunkin Hartley male albino guinea-pig, weighing 300-350 g (Cristol J.P. and Sirois P. Res. Commun. Chem. Pathol., 59, 423 (1988)).

The smooth muscle of guinea-pig ileum exhibits sensitivity to leukotrienes and especially to $LTD_4$, which acts as primary mediator in the inflammatory and allergic response (Carnathan G.W. et al. Agents Actions, 20, 124 (1987)).

The mienteric plexus is extracted from a 2-3 cm segment of the terminal portion of the guinea-pig ileum, previously sacrificed by cervical dislocation. The plexus is put, at a tension of 0.5 g, in a 5 ml organ bath, containing a solution of Tyrode (137 mM NaCl, 2.7 mM KCl, 1.4 mM $CaCl_2$, 0.4 mM $NaH_2PO_4$, 11.9 mM $NaHCO_3$, 0.8 mM $MgSO_4$, 5.5 mM glucose), saturated with carbogen gas (95% $0_2$-5% $CO_2$) at 37°C. The solution also contains indomethacin (3.3 μM) and atropine (0.4 μM) to remove the action of the intrinsic prostaglandins and the cholinergic responses.

After a 45 minute stabilization period a maximum isotonic response is obtained (100% contractile response) adding to the bath chamber the $LTD_4$ agonist (3 nM). This process is repeated until the same contraction response is obtained twice. The isometric measures are made in an isotonic transducer.

After stabilization is restored, the product under test is incubated at different concentrations (dissolved in 0.1% final concentration DMSO) for 2.5 minutes, and after that the contraction with $LTD_4$ is induced again.

The antagonistic activity is expressed as $IC_{50}$, the concentration of compound which reduces by 50% the maximum contraction.

Table 1 shows some of the values of activity found for the compounds of the present invention.

Table 1

| Compound Example N°. | Inhibition of the $[^3H]$-$LTD_4$ receptor binding $K_i$ (nM) | Inhibition of contractions induced in the ileum by $LTD_4$ $IC_{50}$ (nM) |
|---|---|---|
| 1 | 9.2±2 | 13 |
| 2 | 29.5±3 | 66 |
| 3 | 12.0±3 | 18 |
| 5 | 34.0±5 | >100 |
| 6 | 32.7±3 | >100 |
| 10 | 38.5±6.3 | >100 |
| 14 | 68.0±5 | 75 |
| 18 | 134±18 | 100 |
| 20 | 190±21 | 100 |
| 21 | 5.5±0.5 | 6 |
| 23 | 30.0±1.2 | >100 |
| 24 | 28.0±3 | 100 |
| 26 | 1.5±0.2 | 38 |
| 27 | 7.8±2 | 28 |
| 29 | 1.0±0.1 | 8 |
| 30 | 3.4±1.9 | 24 |
| 32 | 44±15 | 100 |
| 33 | 13.1±2 | 87 |

## Claims

1. A compound of formula **I**,

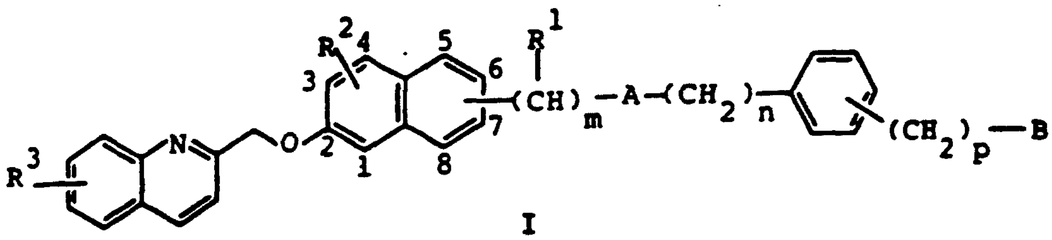

I

wherein:

the substituent containing A is bound to the 6- or 7-position of the 2-naphthol system;
the substituent containing B is bound to the benzene ring at any free position;
-$R^1$ is hydrogen or methyl;
-$R^2$ is hydrogen, fluorine, chlorine or -$OCH_3$, which is bound to the naphthalene system at any positions except the 2- and the one occupied by the other substituent;
-$R^3$ is hydrogen, fluorine, chlorine or bromine;
-A- is a -CO-$NR^4$- or -$NR^4$-CO- group, wherein $R^4$ is hydrogen or methyl;
-B is a 5-tetrazolyl or -$COOR^5$ group, wherein $R^5$ is hydrogen, a ($C_1$-$C_4$)-alkyl or a phenylalkyl group of less than 10 carbon atoms;
m is 0 or 1;
n and p are integers from 0 to 6, with the proviso that n + p is less or equal to 6;
as well as the solvates and pharmaceutically acceptable salts thereof.

2. A compound according to claim 1, wherein $R_2$ is hydrogen, B is a 5-tetrazolyl or $COOR^5$ group, and $R^5$ is hydrogen, methyl, ethyl or benzyl.

3. A compound according to claim 1 or 2, wherein $R^3$ is hydrogen or chlorine, and -A- is -CONH- or -NHCO-.

4. A compound according to any one of claims 1 to 3, wherein the substituent containing A is bound to the 6-position of the 2-naphthol system.

5. A compound according to claim 4, wherein $R^1$ is hydrogen, m is 1, and -A- is -NHCO-.

6. A compound according to claim 4, wherein -A- is -CONH-.

7. A compound according to claim 6, wherein n and p are integers from 0 to 3.

8. A compound according to any one of claims 1 to 3, wherein the substituent containing A is bound to the 7-position of the 2-naphthol system.

9. A compound according to claim 8, wherein $R^1$ is hydrogen, m is 1 and -A- is -CONH-.

10. A compound according to claim 8, wherein m is 0 and A is -CONH-.

11. A compound according to claim 10, wherein n and p are integers from 0 to 3.

12. A compound according to claim 1 selected from the following ones:

N-[4-(1H-5-tetrazolyl)phenylmethyl]-2-[6-(2-quinolinylmethoxy)-2-naphthyl]propanamide;
N-[3-(1H-5-tetrazolyl)phenylmethyl]-2-[6-(2-quinolinylmethoxy)-2-naphthyl]propanamide;
N-[2-(1H-5-tetrazolyl)phenylmethyl]-2-[6-(2-quinolinylmethoxy)-2-naphthyl]propanamide;

N-[4-(1H-5-tetrazolyl)methylphenyl]-2-[6-(2-quinolinylmethoxy)-2-naphthyl]propanamide;
N-[4-(1H-5-tetrazolyl)methylphenyl]-2-[6-(2-quinolinylmethoxy)-2-naphthyl]propanamide (sodium salt);
4-[2-[6-(2-quinolinylmethoxy)-2-naphthyl]propanamido]benzoic acid;
4-[4-[2-[6-(2-quinolinylmethoxy)-2-naphthyl]propanamido]phenyl]butanoic acid;
4-[2-(6-(2-quinolinylmethoxy)-2-naphthyl]propanamidomethyl]benzoic acid;
3-[2-[6-(2-quinolinylmethoxy)-2-naphthyl]propanamidomethyl]benzoic acid;
4-[2-[6-(2-quinolinylmethoxy)-2-naphthyl]propanamido]phenylacetic acid;
3-[2-[6-(2-quinolinylmethoxy)-2-naphthyl]propanamido]phenylacetic acid;
4-[4-[2-[6-[(7-chloro-2-quinolinyl)methoxy]-2-naphthyl]propanamido]phenyl]butanoic acid;
N-[4-(1H-5-tetrazolyl)phenylmethyl]-6-(2-quinolinylmethoxy)-2-naphthaleneacetamide;
4-[4-[[6-(2-quinolinylmethoxy)-2-naphthyl]carboxamido]phenyl]butanoic acid;
N-[3-(1H-5-tetrazolyl)phenylmethyl]-6-(2-quinolinylmethoxy)-2-naphthalenecarboxamide;
4-[4-[[7-(2-quinolinylmethoxy)-2-naphthyl]carboxamido]phenyl]butanoic acid;
N-[4-(1H-5-tetrazolyl)phenylmethyl]-7-(2-quinolinylmethoxy)-2-naphthalenecarboxamide;
4-[2-[[7-(2-quinolinylmethoxy)-2-naphthyl]carboxamido]ethyl]benzoic acid;
N-[4-(1H-5-tetrazolyl)phenylethyl]-7-(2-quinolinylmethoxy)-2-naphthalenecarboxamide;
4-[4-[[6-(2-quinolinylmethoxy)-2-naphthyl]methylaminocarbonyl]phenyl]butanoic acid;
N-[4-(1H-5-tetrazolyl)phenylpropyl]-7-(2-quinolinylmethoxy)-2-naphthalenecarboxamide.

**13.** A process for the preparation of the compounds of general formula **I** of claim 1, and of the pharmaceutically acceptable salts thereof, wherein:

a) when in formula **I** -A- is -CO-NR$^4$-, then a compound of general formula **II,**

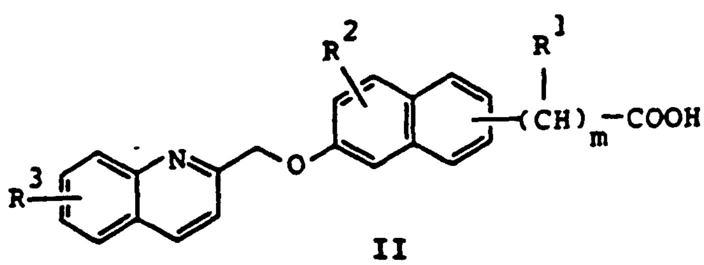

**II**

**II**

wherein R$^1$, R$^2$, R$^3$ and m have the above defined meanings, is reacted with a compound **III**,

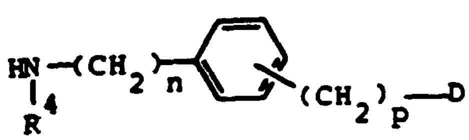

**III**

**III**

wherein R$^4$, n and p have the above defined meanings and D can be equivalent to the group B in **I** or, when B in formula **I** is COOH, then D contains suitable carboxy-protecting group; the reaction between **II** and **III** being carried out in the presence of a carboxy-activating agent and a base, to obtain a compound of formula **IVa**,

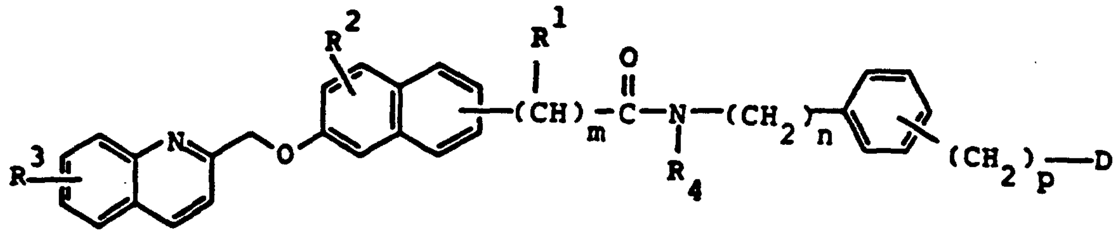

IVa

which is equivalent to **I**, or is converted into **I** by removing of the carboxy-protecting group;
b) when in formula **I** -A- is -$NR^4$-CO-, then a compound of general formula **V**,

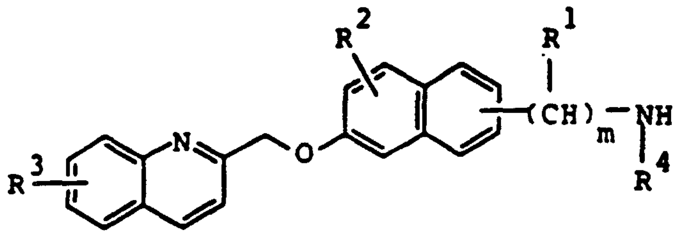

V

V

wherein $R^1$, $R^2$, $R^3$, $R^4$ and m have the above defined meanings, is reacted with a compound **VI**

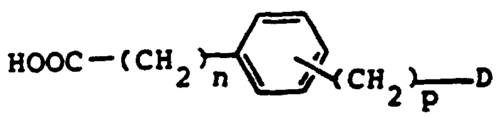

VI

VI

wherein n, p and D have the above defined meanings; the reaction between **V** and **VI** being carried out in the presence of a carboxy-activating agent and a base, to obtain a compound of formula **IVb,**

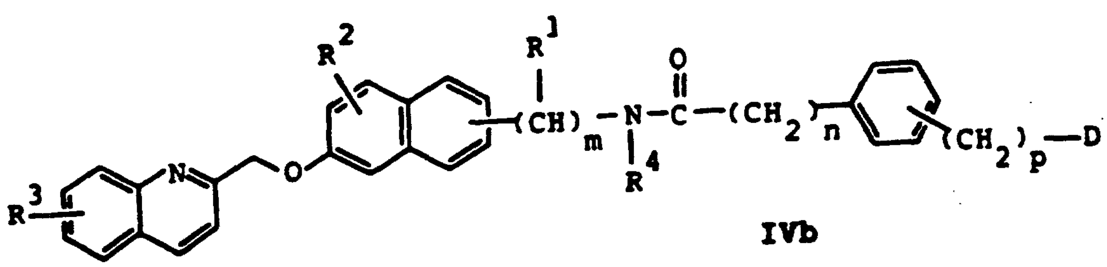

IVb

which coincides with **I**, or is converted into **I** as described above for the compound **IVa**;
c) and, if desired, the compound of general formula **I** is converted into the desired salt, by treatment with a base or a suitable ion-exchanger, according to conventional methods.

**14.** The use of a compound of any one of claims 1 to 12 in the preparation of a medicament for the therapeutical treatment of leukotriene-mediated diseases.

**15.** The use according to claim 14, wherein the leukotriene-mediated diseases are of inflammatory or allergic type.

16. The use according to claim 15, wherein the inflammatory or allergic diseases are: bronchial asthma, allergic rhinitis, allergic conjunctivitis, rheumatoid arthritis, osteoarthritis, tendinitis, bursitis or psoriasis.

17. The use according to claim 14, wherein the leukotriene-mediated diseases are of cardiovascular type.

18. The use according to claim 17, wherein the diseases of cardiovascular type are: cardiac ischemia, cardiac infarction, coronary spasm, cardiac anaphylaxis, cerebral oedema or endotoxic shock.

**Patentansprüche**

1. Eine Verbindung der allgemeinen Formel I

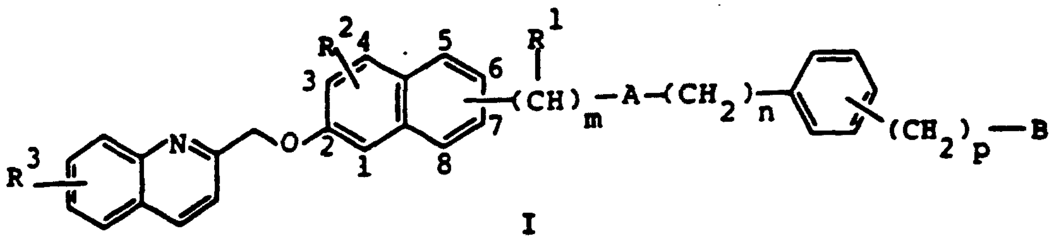

I

worin:

der A-enthaltende Substituent an der 6- oder 7-Position des 2-Naphtholsystems gebunden ist;
der B-enthaltende Substituent an einer beliebigen freien Position am Benzolring gebunden ist;
-$R^1$ ein Wasserstoffatom oder eine Methylgruppe ist;
-$R^2$ ein Wasserstoff-, Fluor- oder Chloratom oder die Gruppe -$OCH_3$ ist, wobei $R^2$ an beliebigen Positionen am Naphthalinsystem gebunden ist, mit Ausnahme der 2-Position und der von dem anderen Substituenten eingenommenen Position;
-$R^3$ ein Wasserstoff-, Fluor-, Chlor- oder Bromatom ist;
-A- eine -CO-$NR^4$- oder -$NR^4$-CO-Gruppe ist, wobei $R^4$ ein Wasserstoffatom oder eine Methylgruppe ist;
-B eine 5-Tetrazolyl- oder -$COOR^5$-Gruppe ist, wobei $R^5$ ein Wasserstoffatom, eine $C_1$-$C_4$-Alkylgruppe oder eine Phenylalkylgruppe mit weniger als 10 Kohlenstoffatomen ist;
m den Wert 0 oder 1 hat;
n und p ganze Zahlen von 0 bis 6 sind, mit der Maßgabe, dass n + p kleiner oder gleich 6 ist; sowie die Solvate und pharmazeutisch verträglichen Salze davon.

2. Verbindung nach Anspruch 1, worin $R^2$ ein Wasserstoffatom, B eine 5-Tetrazolyl- oder $COOR^5$-Gruppe und $R^5$ ein Wasserstoffatom, eine Methyl-, Ethyl- oder Benzylgruppe ist.

3. Verbindung nach Anspruch 1 oder 2, worin $R^3$ ein Wasserstoff- oder Chloratom und -A- die Gruppe -CONH- oder -NHCO- ist.

4. Verbindung nach einem der Ansprüche 1 bis 3, worin der A-enthaltende Substituent an der 6-Position des 2-Naphtholsystems gebunden ist.

5. Verbindung nach Anspruch 4, worin $R^1$ ein Wasserstoffatom ist, m den Wert 1 hat und -A- die -NHCO-Gruppe ist.

6. Verbindung nach Anspruch 4, worin -A- die -CONH-Gruppe ist.

7. Verbindung nach Anspruch 6, worin n und p ganze Zahlen von 0 bis 3 sind.

8. Verbindung nach einem der Ansprüche 1 bis 3, worin der A-enthaltende Substituent an der 7-Position des 2-Naphtholsystems gebunden ist.

**9.** Verbindung nach Anspruch 8, worin $R^1$ ein Wasserstoffatom ist, m den Wert 1 hat und -A- die -CONH-Gruppe ist.

**10.** Verbindung nach Anspruch 8, worin m den Wert 0 hat und -A- die -CONH-Gruppe ist.

**11.** Verbindung nach Anspruch 10, worin n und p ganze Zahlen von 0 bis 3 sind.

**12.** Verbindung nach Anspruch 1, ausgewählt aus den nachstehenden Verbindungen:

N-[4-(1H-5-tetrazolyl)phenylmethyl]-2-[6-(2-chinolinylmethoxy)-2-naphthyl]propanamid;
N-[3-(1H-5-tetrazolyl)phenylmethyl]-2-[6-(2-chinolinylmethoxy)-2-naphthyl]propanamid;
N-[2-(1H-5-tetrazolyl)phenylmethyl]-2-[6-(2-chinolinylmethoxy)-2-naphthyl]propanamid;
N-[4-(1H-5-tetrazolyl)methylphenyl]-2-[6-(2-chinolinylmethoxy)-2-naphthyl]propanamid;
N-[4-(1H-5-tetrazolyl)methylphenyl]-2-[6-(2-chinolinylmethoxy)-2-naphthyl]propanamid (Natriumsalz);
4-[2-[6-(2-Chinoiinyimethoxy)-2-naphthyi]propanamido]benzoesäure;
4-[4-[2-[6-(2-Chinolinylmethoxy)-2-naphthyl]propanamido]phenyl]butansäure;
4-[2-[6-(2-Chinolinylmethoxy)-2-naphthyl]propanamidomethyl]benzoesäure;
3-[2-[6-(2-Chinolinylmethoxy)-2-naphthyl]propanamidomethyl]benzoesäure;
4-[2-[6-(2-Chinolinylmethoxy)-2-naphthyl]propanamido]phenylessigsäure;
3-[2-[6-(2-Chinolinylmethoxy)-2-naphthyl]propanamido]phenylessigsäure;
4-[4-[2-[6-[(7-Chlor-2-chinolinyl)methoxy]-2-naphthyl]propanamido]phenyl]butansäure;
N-[4-(1H-5-tetrazolyl)phenylmethyl]-6-(2-chinolinylmethoxy)-2-naphthalinacetamid;
4-[4-[[6-(2-Chinolinylmethoxy)-2-naphthyl]carboxamido]phenyl]butansäure;
N-[3-(1H-5-tetrazolyl)phenylmethyl]-6-(2-chinolinylmethoxy)-2-naphthalincarboxamid;
4-[4-[[7-(2-Chinolinylmethoxy)-2-naphthyl]carboxamido]phenyl]butansäure;
N-[4-(1H-5-tetrazolyl)phenylmethyl]-7-(2-chinolinylmethoxy)-2-naphthalincarboxamid;
4-[2-[[7-(2-chinolinylmethoxy)-2-naphthyl]carboxamido]ethyl]benzoesäure;
N-[4-(1H-5-tetrazolyl)phenylethyl]-7-(2-chinolinylmethoxy)-2-naphthalincarboxamid;
4-[4-[[6-(2-Chinolinylmethoxy)-2-naphthyl]methylaminocarbonyl]phenyl]butansäure;
N-[4-(1H-5-tetrazolyl)phenylpropyl]-7-(2-chinolinylmethoxy)-2-naphthalincarboxamid.

**13.** Ein Verfahren zur Herstellung der Verbindungen der allgemeinen Formel I nach Anspruch 1 und deren pharmazeutisch verträglichen Salzen, wobei:

a) wenn in der allgemeinen Formel I -A- die Gruppe -CO-$NR^4$- ist, eine Verbindung der allgemeinen Formel II

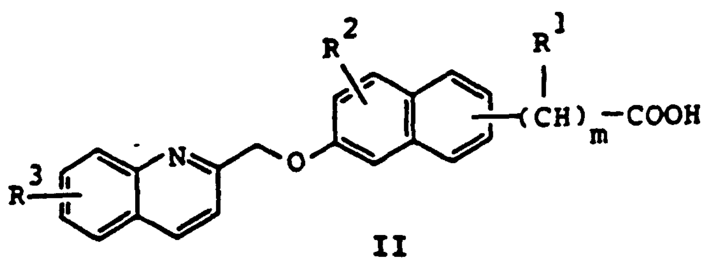

II

II

worin $R^1$, $R^2$, $R^3$ und m die vorstehend angegebenen Bedeutungen haben, mit einer Verbindung III umgesetzt wird

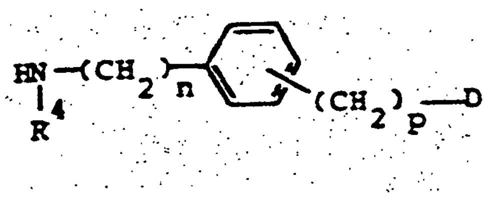

III

worin $R^4$, n und p die vorstehend angegebenen Bedeutungen haben und D zur Gruppe B in I äquivalent sein kann oder D enthält eine geeignete Carboxylschutzgruppe, wenn B in der allgemeinen Formel 1 die Gruppe COOH ist, wobei die Umsetzung von II und III in Gegenwart eines Carboxyl-Aktivierungsmittels und einer Base durchgeführt wird, um eine Verbindung der allgemeinen Formel IVa zu erhalten

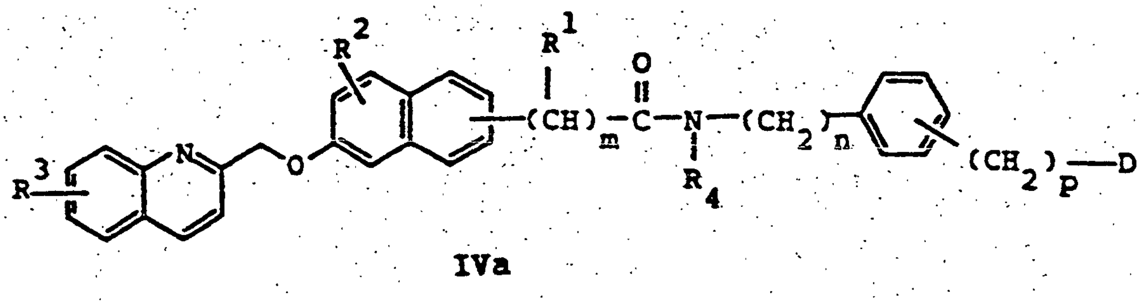

IVa

die äquivalent zu I ist, oder durch Entfernen der Carboxylschutzgruppe in I umgewandelt wird;

b) wenn in der allgemeinen Formel I -A- die Gruppe -$NR^4$-CO- ist, eine Verbindung der allgemeinen Formel V

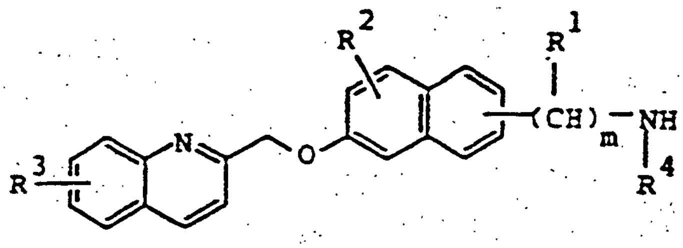

V

worin $R^1$, $R^2$, $R^3$, $R^4$ und m die vorstehend angegebenen Bedeutungen haben, mit einer Verbindung VI umgesetzt wird

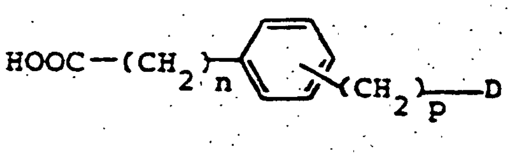

VI

worin n, p und D die vorstehend angegebenen Bedeutungen haben, wobei die Umsetzung von V und VI in Gegenwart eines Carboxyl-Aktivierungsmittels und einer Base durchgeführt wird, um eine Verbindung der allgemeinen Formel IVb zu erhalten

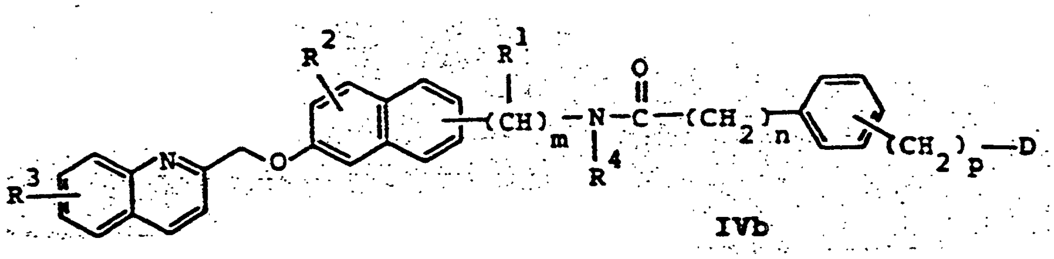

IVb

die äquivalent zu I ist, oder wie vorstehend für die Verbindung IVa beschrieben in I umgewandelt wird;

c) und wobei gegebenenfalls die Verbindung der allgemeinen Formel I in das gewünschte Salz umgewandelt wird, und zwar durch Behandlung mit einer Base oder einem geeigneten Ionenaustauscher gemäß herkömmlicher Verfahren.

**14.** Die Verwendung einer Verbindung nach einem der Ansprüche 1 bis 12 bei der Herstellung eines Medikaments für die therapeutische Behandlung Leukotrien-vermittelter Erkrankungen.

**15.** Verwendung nach Anspruch 14, wobei die Leukotrien-vermittelten Erkrankungen Entzündungs- oder Allergieerkrankungen sind.

**16.** Verwendung nach Anspruch 15, wobei die Entzündungs- oder Allergieerkrankungen Bronchialasthma, allergische Rhinitis, allergische Konjunktivitis, rheumatoide Arthritis, Osteoarthrose, Tendinitis, Bursitis oder Psoriasis sind.

**17.** Verwendung nach Anspruch 14, wobei die Leukotrien-vermittelten Erkrankungen Kardiovaskulärerkrankungen sind.

**18.** Verwendung nach Anspruch 17, wobei die Kardiovaskulärerkrankungen Herzischämie, Herzinfarkt, Koronarspasmus, Herzanaphylaxie, Zerebralödem oder Endotoxinschock sind.

**Revendications**

**1.** Composé de formule I,

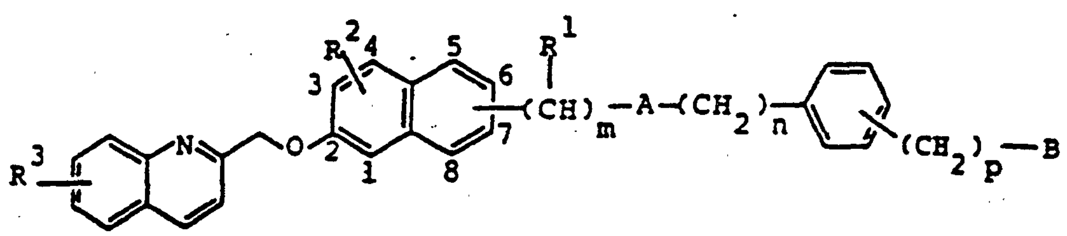

I

dans laquelle :

le substituant contenant A est lié à la position 6 ou 7 du système 2-naphtol;
le substituant contenant B est lié au noyau benzène en n'importe quelle position libre ;
-$R^1$ est hydrogène ou méthyle ;
-$R^2$ est hydrogène, fluor, chlore ou -$OCH_3$, qui est lié au système naphtalène en n'importe quelle position excepté en position 2 et celle occupée par l'autre substituant ;
-$R^3$ est hydrogène, fluor, chlore ou brome ;
-A- est un groupe -CO-$NR^4$- ou -$NR^4$-CO-, où $R^4$ est hydrogène ou méthyle ;
-B est un groupe 5-tétrazolyle ou -$COOR^5$, où $R^5$ est hydrogène, un groupe alkyle en $C_1$-$C_4$ ou un groupe phénylalkyle ayant moins de 10 atomes de carbone ;
m est 0 ou1 ;
n et p sont des nombres entiers de 0 à 6, à la condition que n + p soit inférieur ou égal à 6;

ainsi que les solvates et les sels pharmaceutiquement acceptables de celui-ci.

**2.** Composé selon la revendication 1, dans lequel $R_2$ est hydrogène, B est un groupe 5-tétrazolyle ou -$COOR^5$, et $R^5$ est hydrogène, méthyle, éthyle ou benzyle.

**3.** Composé selon la revendication 1 ou 2, dans lequel $R^3$ est hydrogène ou chlore, et -A- est -CONH- ou -NHCO-.

**4.** Composé selon l'une des revendications 1 à 3, dans lequel le substituant contenant A est lié à la position 6 du système 2-naphtol.

**5.** Composé selon la revendication 4, dans lequel $R^1$ est hydrogène, m est 1, et -A- est -NHCO-.

**6.** Composé selon la revendication 4, dans lequel -A- est -CONH-.

**7.** Composé selon la revendication 6, dans lequel n et p sont des nombres entiers de 0 à 3.

**8.** Composé selon l'une des revendications 1 à 3, dans lequel le substituant A est lié à la position 7 du système 2-naphtol.

**9.** Composé selon la revendication 8, dans lequel $R^1$ est hydrogène, m est 1 et -A- est -CONH-.

**10.** Composé selon la revendication 8, dans lequel m est 0 et A est -CONH-.

**11.** Composé selon la revendication 10, dans lequel n et p sont des nombres entiers de 0 à 3.

**12.** Composé selon la revendication 1 choisi parmi les composés suivants :

N-[4-(1H-5-tétrazolyl)phénylméthyl]-2-[6-(2-quinolinyl-méthoxy)-2-naphtyl]propanamide ;
N-[3-(1H-5-tétrazolyl)phénylméthyl]-2-[6-(2-quinolinyl-méthoxy)-2-naphtyl]propanamide ;
N-[2-(1H-5-tétrazolyl)phénylméthyl]-2-[6-(2-quinolinyl-méthoxy)-2-naphtyl]propanamide ;
N-[4-(1H-5-tétrazolyl)méthylphényl]-2-[6-(2-quinolinyl-méthoxy)-2-naphtyl]propanamide ;
(Sel de sodium) de N-[4-(1H-5-tétrazolyl)méthylphényl]-2-[6-(2-quinolinyl-méthoxy)2-naphtyl]propanamide ;
Acide 4-[2-[6-(2-quinolinylméthoxy)-2-naphtyl]propanamido]benzoïque ;
Acide 4-[4-[2-[6-(2-quinolinylméthoxy)-2-naphtyl]propanamido]phényl]butanoïque ;
Acide 4-[2-[6-(2-quinolinylméthoxy)-2-naphtyl]propanamidométhyl]benzoïque ;
Acide 3-[2-[6-(2-quinolinylméthoxy)-2-naphtyl]propanamidométhyl]benzoïque;
Acide 4-[2-[6-(2-quinolinylméthoxy)-2-naphtyl]propanamido]phénylacétique ;
Acide 3-[2-[6-(2-quinolinylméthoxy)-2-naphtyl]propanamido]phénylacétique ;
Acide 4-[4-[2-[6-[(7-chloro-2-quinolinyl)méthoxy]-2-naphtyl]propanamido]phényl]butanoïque ;
N-[4-(1H-5-tétrazolyl)phénylméthyl]-6-(2-quinolinylméthoxy)-2-naphtalèneacétamide ;
Acide 4-[4-[[6-(2-quinolinylméthoxy)-2-naphtyl]carboxamido]phényl]butanoïque ;
N-[3-(1H-5-tétrazolyl)phénylméthyl]-6-(2-quinolinylméthoxy)-2-naphtalènecarboxamide ;
Acide 4-[4-[[7-(2-quinolinylméthoxy)-2-naphtyl]carboxamido]phényl]butanoïque ;
N-[4-(1H-5-tétrazolyl)phénylméthyl]-7-(2-quinolinylméthoxy)-2-naphtalènecarboxamide ;
Acide 4-[2-[[7-(2-quinolinylméthoxy)-2-naphtyl]carboxamido]éthyl]benzoïque ;
N-[4-(1H-5-tétrazolyl)phényléthyl]-7-(2-quinolinylméthoxy)-2-naphtalènecarboxamide ;
Acide 4-[4-[[6-(2-quinolinylméthoxy)-2-naphtyl]méthylaminocarbonyl]phényl]butanoïque ;
N-[4-(1H-5-tétrazolyl)phénylpropyl]-7-(2-quinolinylméthoxy)-2-naphtalènecarboxamide .

**13.** Procédé pour la préparation des composés de formule générale I de la revendication 1 , et des sels pharmaceutiquement acceptables de ceux-ci, dans lequel :

a) quand dans la formule I, -A- est -CO-NR$^4$-, alors un composé de formule générale II,

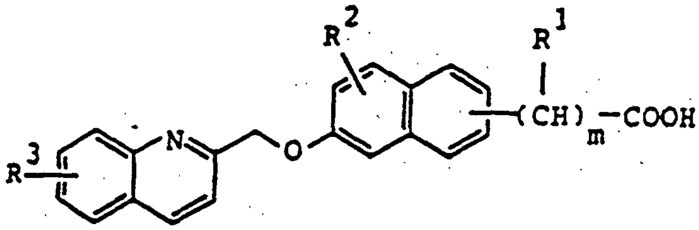

II

dans laquelle $R^1$, $R^2$, $R^3$ et m ont les significations définies ci-dessus, est mis à réagir avec un composé III,

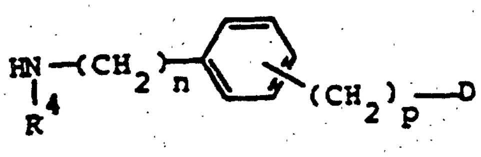

III

dans laquelle $R^4$, n et p ont les significations définies ci-dessus et D peut être équivalent au groupe B dans I ou, quand B dans la formule I est COOH, alors D contient un groupe approprié de protection de carboxy ; la réaction entre II et III étant effectuée en présence d'un agent d'activation de carboxy et d'une base, pour obtenir un composé de formule IVa,

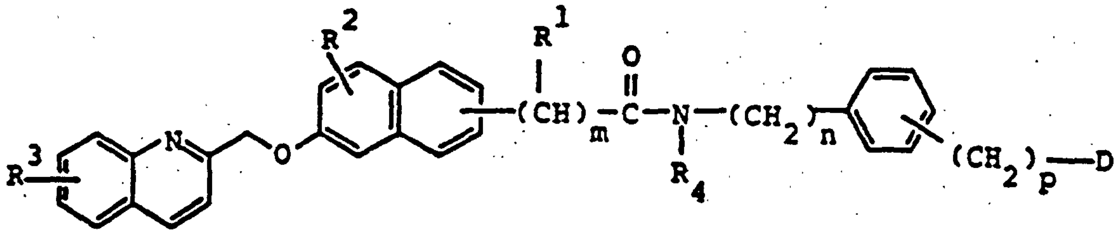

IVa

qui est équivalent à I, ou qui est converti en I par élimination du groupe de protection de carboxy ;
b) quand dans la formule I, -A- est $-NR^4-CO-$, alors un composé de formule générale V,

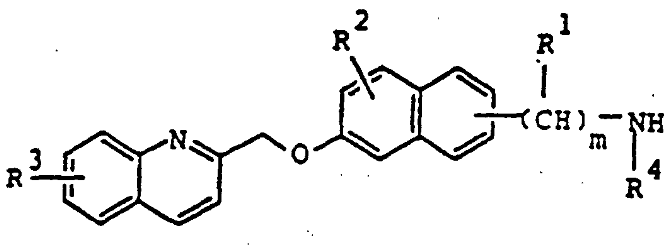

V

dans laquelle $R^1$, $R^2$, $R^3$, $R^4$ et m ont les significations définies ci-dessus, est mis à réagir avec un composé VI

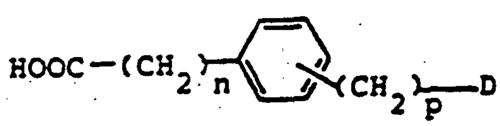

VI

dans lequel n, p et D ont les significations définies ci-dessus ; la réaction entre V et VI étant effectuée en présence d'un agent d'activation de carboxy et d'une base, pour obtenir un composé de formule IVb,

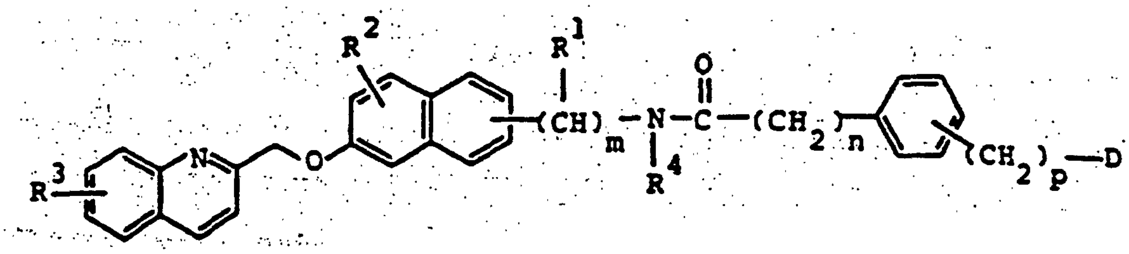

IVb

qui coïncide avec I, ou qui est converti en I comme cela est décrit ci-dessus pour le composé IVa ;
c) et, si souhaité, le composé de formule générale I est transformé en le sel souhaité, par traitement avec une base ou avec un échangeur d'ions approprié, selon des procédés conventionnels.

**14.** Utilisation d'un composé de l'une des revendications 1 à 12 dans la préparation d'un médicament pour le traitement thérapeutique des maladies faisant intervenir un médiateur du type leucotriène.

**15.** Utilisation selon la revendication 14, dans laquelle les maladies faisant intervenir un médiateur du type leucotriène sont soit du type inflammatoire soit du type allergique.

**16.** Utilisation selon la revendication 15, dans laquelle les maladies inflammatoires ou allergiques sont : l'asthme bronchique, la rhinite allergique, la conjonctivite allergique, le rhumatisme articulaire, l'ostéo-arthrite, la tendinite, la bursite ou le psoriasis.

**17.** Utilisation selon la revendication 14, dans laquelle les maladies faisant intervenir un médiateur du type leucotriène sont du type cardiovascuaire.

**18.** Utilisation selon la revendication 17, dans laquelle les maladies du type cardiovasculaire sont : l'ischémie cardiaque, l'infarctus cardiaque, le spasme coronaire, l'anaphylaxie cardiaque, l'oedème cérébral ou le choc endotoxique.